# EUROPEAN PATENT APPLICATION

(11) **EP 4 410 840 A1**
(43) Date of publication of application: **07.08.2024**
(21) Application number: 22875039.4
(22) Date of filing: 29.09.2022
(51) Int. Cl.: C07K 16/46, C07K 16/22, C07K 16/28, A61K 39/395, A61K 39/00, A61P 35/00, C12N 15/62, C12N 15/63

(54) **BISPECIFIC ANTIBODY AND APPLICATION THEREOF**

(30) Priority: 30.09.2021 CN 202111162303
(71) Applicant: Betta Pharmaceuticals Co., Ltd, Yuhang Hangzhou Zhejiang 311100 (CN)
(72) Inventor: XU, Wenxin, Hangzhou, Zhejiang 311100 (CN); FANG, Xuefei, Hangzhou, Zhejiang 311100 (CN); XU, Deyu, Hangzhou, Zhejiang 311100 (CN); XU, Jieying, Hangzhou, Zhejiang 311100 (CN); DING, Lieming, Hangzhou, Zhejiang 311100 (CN)
(74) Representative: J A Kemp LLP
(86) International application number: PCT/CN2022/122367
(87) International publication number: WO 2023/051656

(57) **Abstract**

Provided are a bispecific antibody and use thereof. The bispecific antibody provided can specifically bind to TGF-β, GARP, or GARP-TGF-β complex individually or simultaneously, and can also specifically bind to PD-L1. The bispecific antibody provided exhibits high affinity and specificity for the antigens, has a tumor-killing effect, and can be applied to the treatment of tumors.

## Description

### CROSS-REFERENCE TO RELATED APPLICATIONS

The present invention claims priority to Chinese Patent Application No. 202111162303.X filed on Sept. 30, 2021, which is incorporated herein by reference in its entirety or in part.

### TECHNICAL FIELD

The present invention relates to the field of biopharmaceutics and in particular to a bispecific antibody and use thereof.

### BACKGROUND

Transforming growth factor-β (TGF-β), as a TGF-β superfamily, is expressed in a variety of immune cells, such as regulatory T cells (Tregs), DC cells, CD3+ T cells, M2 macrophages, etc., and regulates cell growth and differentiation. In the tumor microenvironment, the activity of immune cells is often influenced by TGF-β in the environment, resulting in immunosuppression. Precisely due to the effectiveness and diversity of TGF-β, subtypes such as TGF-β1, -β2, and -β3 among the members of the family are generated in latent, inactive forms and require tightly regulated extracellular activation steps to gain the ability to bind to their receptors (Cold Spring Harb. Perspect. Biol. 2016; 8(a022103), Cytokine Growth Factor Rev. 2013; 24:355-372). TGF-β1 is a major subtype expressed by immune cells such as Tregs. It is by producing active TGF-β that Tregs mediate immunosuppressive effects, thereby preventing the development of diseases such as autoimmune diseases. However, malignancies can utilize this mechanism to release a large amount of TGF-β, helping cancer cells divide rapidly and, through Tregs, suppressing the killing of cancer cells by other immune cells.

Glycoprotein A repetitions predominant (GARP), a type I transmembrane cell surface receptor that binds to latent transforming growth factor β (TGF-β), is mainly activated on the surfaces of Tregs and B cells and in platelets and is highly expressed in tumors of the human breast, lungs, and colon. GARP can be combined with latent TGF-β (latency associated peptide, LAP) to form a complex (Proc Natl Acad Sci USA. 2009; 106(32): 13445-50), can adjust the capacity of the membrane to bind to latent TGF-β, and can bind to αVβ8 integrin or αVβ6 integrin to release TGF-β from the cell surface and activate it.

Given that high GARP expression provides tumors with activation and storage of TGF-β necessary for growth and also strengthens the suppressive phenotype of Tregs and maintains Treg-mediated peripheral tolerance, GARP inhibitors have gradually emerged as a key player in the treatment of tumors. The development of GARP-targeting antibodies is advancing rapidly; for example, ARGX-115, the first GARP inhibitor to enter clinical trials, takes GARP as a target and inhibits the release of active TGF-β and the immunosuppressive activity of Tregs, thereby reactivating immune responses to cancer cells and inhibiting disease progression. DS-1055 (a monoclonal antibody designed against GARP), developed by Daiichi Sankyo, is also undergoing clinical trials for recurrent/refractory advanced or metastatic head and neck, gastric, and esophageal cancers.

Bispecific antibodies (BsAbs) are antibodies that can specifically bind to two antigens or epitopes simultaneously. Since the concept of BsAbs was first proposed by Nisonoff and his collaborators in 1960, the development of bispecific antibodies has advanced rapidly with the maturation of genetic engineering techniques for antibodies. As BsAbs can target multiple antigens or epitopes, they have more advantages than monoclonal antibodies and have also gradually shown higher therapeutic effects than combination therapy with monoclonal antibodies. For example, specific immune effector cells can be redirected to adjacent tumor cells to enhance tumor killing; moreover, the specificity of binding can be increased through the interaction between two different cell surface antigens; meanwhile, compared to combinations with single antibodies, BsAbs enable reductions in development costs and clinical trials. Bispecific antibodies have now become a popular research topic in the area of antibody engineering and have wide application prospects in tumor treatment, autoimmune diseases, etc. Research on bispecific antibodies has been conducted against different targets by several Chinese and foreign pharmaceutical companies.

However, bispecific antibodies involve the interaction between two or more entities, making them more complex than monoclonal antibodies. There is still a need for further improvement in the development of bispecific antibodies against different targets.

### SUMMARY

The present invention provides a bispecific antibody and use thereof in the treatment of disease. According to the present invention, the release of active TGF-β and the immunosuppressive function of Tregs are inhibited by targeting GARP, TGF-β, and/or GARP-TGF-β complex, so that immune responses to cancer cells are reactivated and disease progression is inhibited. Moreover, the bispecific antibody provided can also specifically bind to PD-L1 and can improve PD-1/PD-L1 drug-resistant anti-tumor activity.

A first aspect of the present invention provides a bispecific antibody, wherein the bispecific antibody comprises a first antigen-binding moiety and a second antigen-binding moiety; the first antigen-binding moiety comprises a heavy chain variable region, and the heavy chain variable region comprises HCDR sequences set forth in SEQ ID NOs: 1, 2, and 3, or sequences that have one or two amino acid substitutions, deletions, or additions compared to the HCDR sequences set forth in SEQ ID NOs: 1, 2, and 3; or comprises HCDR sequences set forth in SEQ ID NOs: 4, 5, and 6, or sequences that have one or two amino acid substitutions, deletions, or additions compared to the HCDR sequences set forth in SEQ ID NOs: 4, 5, and 6; or comprises HCDR sequences set forth in SEQ ID NOs: 7, 8, and 9, or sequences that have one or two amino acid substitutions, deletions, or additions compared to the HCDR sequences set forth in SEQ ID NOs: 7, 8, and 9; or comprises HCDR sequences set forth in SEQ ID NOs: 10, 11, and 12, or sequences that have one or two amino acid substitutions, deletions, or additions compared to the HCDR sequences set forth in SEQ ID NOs: 10, 11, and 12; or comprises HCDR sequences set forth in SEQ ID NOs: 13, 14, and 15, or sequences that have one or two amino acid substitutions, deletions, or additions compared to the HCDR sequences set forth in SEQ ID NOs: 13, 14, and 15; or comprises HCDR sequences set forth in SEQ ID NOs: 16, 17, and 18, or sequences that have one or two amino acid substitutions, deletions, or additions compared to the HCDR sequences set forth in SEQ ID NOs: 16, 17, and 18; the first antigen-binding moiety further comprises a light chain variable region, and the light chain variable region comprises LCDR sequences set forth in SEQ ID NOs: 19, 20, and 21, or sequences that have one or two amino acid substitutions, deletions, or additions compared to the LCDR sequences set forth in SEQ ID NOs: 19, 20, and 21; or comprises LCDR sequences set forth in SEQ ID NOs: 22, 23, and 24, or sequences that have one or two amino acid substitutions, deletions, or additions compared to the LCDR sequences set forth in SEQ ID NOs: 22, 23, and 24; or comprises LCDR sequences set forth in SEQ ID NOs: 25, 20, and 26, or sequences that have one or two amino acid substitutions, deletions, or additions compared to the LCDR sequences set forth in SEQ ID NOs: 25, 20, and 26; or comprises LCDR sequences set forth in SEQ ID NOs: 27, 28, and 29, or sequences that have one or two amino acid substitutions, deletions, or additions compared to the LCDR sequences set forth in SEQ ID NOs: 27, 28, and 29; or comprises LCDR sequences set forth in SEQ ID NOs: 30, 31, and 32, or sequences that have one or two amino acid substitutions, deletions, or additions compared to the LCDR sequences set forth in SEQ ID NOs: 30, 31, and 32; or comprises LCDR sequences set forth in SEQ ID NOs: 33, 34, and 35, or sequences that have one or two amino acid substitutions, deletions, or additions compared to the LCDR sequences set forth in SEQ ID NOs: 33, 34, and 35;

the first antigen-binding moiety is capable of binding to TGF-β, GARP, or GARP-TGF-β complex, and the second antigen-binding moiety is capable of binding to PD-L1; the HCDR sequences and the LCDR sequences are obtained based on the IMGT definition scheme.

The bispecific antibody provided has a first antigen-binding moiety and a second antigen-binding moiety, wherein the first antigen-binding moiety is capable of specifically targeting one, two, or three of GARP, TGF-β or GARP-TGF-β complex and activating immune cell responses; the second antigen-binding moiety is capable of specifically targeting PD-L1, blocking the PD-1/PD-L1 signaling pathway, eliminating immunosuppression, and restoring the immune killing ability of the body. The bispecific antibody provided has been shown to exhibit an excellent therapeutic effect on tumors.

A second aspect of the present invention provides a bispecific antibody, comprising a first antigen-binding moiety and a second antigen-binding moiety, wherein the first antigen-binding moiety comprises a heavy chain variable region and a light chain variable region; the heavy chain variable region comprises complementarity-determining regions HCDR1, HCDR2, and HCDR3, and the light chain variable region comprises complementarity-determining regions LCDR1, LCDR2, and LCDR3, wherein the HCDR1 comprises the sequences set forth in SEQ ID NO: 1 or 4 or 7 or 10 or 13 or 16, the HCDR2 comprises the sequences set forth in SEQ ID NO: 2 or 5 or 8 or 11 or 14 or 17, the HCDR3 comprises the sequences set forth in SEQ ID NO: 3 or 6 or 9 or 12 or 15 or 18, the LCDR1 comprises the sequences set forth in SEQ ID NO: 19 or 22 or 25 or 27 or 30 or 33, the LCDR2 comprises the sequences set forth in SEQ ID NO: 20 or 23 or 28 or 31 or 34, and the LCDR3 comprises the sequences set forth in SEQ ID NO: 21 or 24 or 26 or 29 or 32 or 35; the first antigen-binding moiety is capable of binding to TGF-β, GARP, or GARP-TGF-β complex, and the second antigen-binding moiety is capable of binding to PD-L1; the HCDR1, HCDR2, and HCDR3 sequences and the LCDR1, LCDR2, and LCDR3 sequences are obtained based on the IMGT definition scheme.

A third aspect of the present invention provides a bispecific antibody, comprising a first antigen-binding moiety and a second antigen-binding moiety, wherein the first antigen-binding moiety is capable of binding to GARP, TGFβ, and/or GARP-TGF-β complex;

the second antigen-binding moiety comprises a CDR1, a CDR2, and a CDR3, wherein:
(a) the CDR1 comprises a sequence selected from the group consisting of SEQ ID NOs: 60, 63, and 66, or sequences that have one or two amino acid substitutions, deletions, or additions compared to SEQ ID NOs: 60, 63, and 66;
(b) the CDR2 comprises a sequence selected from the group consisting of SEQ ID NOs: 61, 64, and 67, or sequences that have one or two amino acid substitutions, deletions, or additions compared to SEQ ID NOs: 61, 64, and 67;
(c) the CDR3 comprises a sequence selected from the group consisting of SEQ ID NOs: 62, 65, 68, and 69, or sequences that have one, two, or three amino acid substitutions, deletions, or additions compared to SEQ ID NOs: 62, 65, 68, and 69;
   the CDR1, CDR2, and CDR3 sequences are obtained based on the IMGT definition scheme.

A fourth aspect of the present invention provides a bispecific antibody, comprising a first antigen-binding moiety and a second antigen-binding moiety, wherein the first antigen-binding moiety comprises a heavy chain variable region and a light chain variable region; the heavy chain variable region comprises HCDR sequences set forth in SEQ ID NOs: 1, 2, and 3, or comprises HCDR sequences set forth in SEQ ID NOs: 4, 5, and 6, or comprises HCDR sequences set forth in SEQ ID NOs: 7, 8, and 9, or comprises HCDR sequences set forth in SEQ ID NOs: 10, 11, and 12, or comprises HCDR sequences set forth in SEQ ID NOs: 13, 14, and 15, or comprises HCDR sequences set forth in SEQ ID NOs: 16, 17, and 18; the light chain variable region comprises LCDR sequences set forth in SEQ ID NOs: 19, 20, and 21, or comprises LCDR sequences set forth in SEQ ID NOs: 22, 23, and 24, or comprises LCDR sequences set forth in SEQ ID NOs: 25, 20, and 26, or comprises LCDR sequences set forth in SEQ ID NOs: 27, 28, and 29, or comprises LCDR sequences set forth in SEQ ID NOs: 30, 31, and 32, or comprises LCDR sequences set forth in SEQ ID NOs: 33, 34, and 35; the second antigen-binding moiety comprises a CDR1, a CDR2, and a CDR3; the CDR1 comprises the sequence set forth in SEQ ID NO: 60 or 63 or 66, the CDR2 comprises the sequence set forth in SEQ ID NO: 61 or 64 or 67, and the CDR3 comprises the sequence set forth in SEQ ID NO: 62 or 65 or 68 or 69.

A fifth aspect of the present invention provides a bispecific antibody, comprising a first antigen-binding moiety and a second antigen-binding moiety, wherein the first antigen-binding moiety comprises: the HCDR1, HCDR2, and HCDR3 sequences of a heavy chain variable region set forth in SEQ ID NO: 36 or 37 or 38 or 39 or 40 or 41 or 42 or 43 or 44 or 45 or 46 or 47 and the LCDR1, LCDR2, and LCDR3 sequences of a light chain variable region set forth in SEQ ID NO: 48 or 49 or 50 or 51 or 52 or 53 or 54 or 55 or 56 or 57 or 58 or 59; the first antigen-binding moiety is capable of binding to TGF-β, GARP, or GARP-TGF-β complex, and the second antigen-binding moiety is capable of binding to PD-L1.

A sixth aspect of the present invention provides a bispecific antibody, comprising a first antigen-binding moiety that specifically binds to TGFβ, GARP, and/or GARP-TGFβ complex, and a second antigen-binding moiety that specifically binds to PD-L1; further comprising a first heavy chain constant domain CH1 and a light chain constant domain CL, wherein the first antigen-binding moiety is covalently linked to the first heavy chain constant domain CH1 and the light chain constant domain CL; and further comprising an Fc region, wherein the Fc region is linked to the first heavy chain constant domain CH1 by a hinge region, and the second antigen-binding moiety is covalently linked to the Fc region and/or the light chain constant domain CL by a linker.

A seventh aspect of the present invention provides a monoclonal antibody or an antigen-binding fragment, comprising a heavy chain variable region, wherein the heavy chain variable region comprises HCDR sequences set forth in SEQ ID NOs: 1, 2, and 3, or sequences that have one, two, or three amino acid substitutions, deletions, or additions compared to the HCDR sequences set forth in SEQ ID NOs: 1, 2, and 3; or comprises HCDR sequences set forth in SEQ ID NOs: 4, 5, and 6, or sequences that have one, two, or three amino acid substitutions, deletions, or additions compared to the HCDR sequences set forth in SEQ ID NOs: 4, 5, and 6; or comprises HCDR sequences set forth in SEQ ID NOs: 7, 8, and 9, or sequences that have one, two, or three amino acid substitutions, deletions, or additions compared to the HCDR sequences set forth in SEQ ID NOs: 7, 8, and 9; or comprises HCDR sequences set forth in SEQ ID NOs: 10, 11, and 12, or sequences that have one, two, or three amino acid substitutions, deletions, or additions compared to the HCDR sequences set forth in SEQ ID NOs: 10, 11, and 12; or comprises HCDR sequences set forth in SEQ ID NOs: 13, 14, and 15, or sequences that have one, two, or three amino acid substitutions, deletions, or additions compared to the HCDR sequences set forth in SEQ ID NOs: 13, 14, and 15; or comprises HCDR sequences set forth in SEQ ID NOs: 16, 17, and 18, or sequences that have one, two, or three amino acid substitutions, deletions, or additions compared to the HCDR sequences set forth in SEQ ID NOs: 16, 17, and 18.

An eighth aspect of the present invention provides a monoclonal antibody or an antigen-binding fragment, comprising a heavy chain variable region and a light chain variable region, wherein the heavy chain variable region and the light chain variable region have: (1) HCDR sequences set forth in SEQ ID NOs: 1, 2, and 3 and LCDR sequences set forth in SEQ ID NOs: 19, 20, and 21; or (2) HCDR sequences set forth in SEQ ID NOs: 4, 5, and 6 and LCDR sequences set forth in SEQ ID NOs: 22, 23, and 24; or (3) HCDR sequences set forth in SEQ ID NOs: 7, 8, and 9 and LCDR sequences set forth in SEQ ID NOs: 25, 20, and 26; or (4) HCDR sequences set forth in SEQ ID NOs: 10, 11, and 12 and LCDR sequences set forth in SEQ ID NOs: 27, 28, and 29; or (5) HCDR sequences set forth in SEQ ID NOs: 13, 14, and 15 and LCDR sequences set forth in SEQ ID NOs: 30, 31, and 32; or (6) HCDR sequences set forth in SEQ ID NOs: 16, 17, and 18 and LCDR sequences set forth in SEQ ID NOs: 33, 34, and 35.

A ninth aspect of the present invention provides a monoclonal antibody or an antigen-binding fragment, comprising a heavy chain variable region and a light chain variable region, wherein the heavy chain variable region comprises complementarity-determining regions HCDR1, HCDR2, and HCDR3, and the light chain variable region comprises complementarity-determining regions LCDR1, LCDR2, and LCDR3, wherein the HCDR1 comprises the sequence set forth in SEQ ID NO: 1 or 4 or 7 or 10 or 13 or 16, the HCDR2 comprises the sequence set forth in SEQ ID NO: 2 or 5 or 8 or 11 or 14 or 17, the HCDR3 comprises the sequence set forth in SEQ ID NO: 3 or 6 or 9 or 12 or 15 or 18, the LCDR1 comprises the sequence set forth in SEQ ID NO: 19 or 22 or 25 or 27 or 30 or 33, the LCDR2 comprises the sequence set forth in SEQ ID NO: 20 or 23 or 28 or 31 or 34, and the LCDR3 comprises the sequence set forth in SEQ ID NO: 21 or 24 or 26 or 29 or 32 or 35.

A tenth aspect of the present invention provides a monoclonal antibody or an antigen-binding fragment, comprising the HCDR1, HCDR2, and HCDR3 sequences of a heavy chain variable region set forth in SEQ ID NO: 36 or 37 or 38 or 39 or 40 or 41 or 42 or 43 or 44 or 45 or 46 or 47 and the LCDR1, LCDR2, and LCDR3 sequences of a light chain variable region set forth in SEQ ID NO: 48 or 49 or 50 or 51 or 52 or 53 or 54 or 55 or 56 or 57 or 58 or 59.

An eleventh aspect of the present invention provides a polynucleotide, wherein the polynucleotide encodes the bispecific antibody according to any of the first to sixth aspects described above, or encodes the monoclonal antibody or the antigen-binding fragment according to any of the seventh to tenth aspects.

A twelfth aspect of the present invention provides a construct, comprising the polynucleotide according to the eleventh aspect described above.

A thirteenth aspect of the present invention provides a host cell, comprising the polynucleotide according to the eleventh aspect described above or the construct according to the twelfth aspect.

A fourteenth aspect of the present invention provides a pharmaceutical composition, comprising the bispecific antibody according to any of the first to sixth aspects described above, or the monoclonal antibody or the antigen-binding fragment according to any of the seventh to tenth aspects, and a pharmaceutically acceptable carri er.

A fifteenth aspect of the present invention provides an antibody conjugate, comprising the bispecific antibody according to any of the first to sixth aspects described above, or the monoclonal antibody or the antigen-binding fragment according to any of the seventh to tenth aspects, and a functional small molecule linked to the bispecific antibody or the monoclonal antibody or the antigen-binding fragment.

A sixteenth aspect of the present invention provides a kit, wherein the kit comprises the bispecific antibody according to any of the examples of the first to sixth aspects described above, or the monoclonal antibody or the antigen-binding fragment according to any of the seventh to tenth aspects.

A seventeenth aspect of the present invention provides a method for producing the bispecific antibody described above or producing the monoclonal antibody or the antigen-binding fragment described above, comprising: culturing the host cell according to the thirteenth aspect, and collecting from the culture the bispecific antibody or the monoclonal antibody or the antigen-binding fragment.

An eighteenth aspect of the present invention provides a method for preventing and/or treating disease, comprising: administering to a subject in need thereof an effective amount of the bispecific antibody according to any of the first to sixth aspects described above, or the monoclonal antibody or the antigen-binding fragment according to any of the seventh to tenth aspects described above, or the pharmaceutical composition according to the fourteenth aspect, or the antibody conjugate according to the fifteenth aspect.

A nineteenth aspect of the present invention provides use of the bispecific antibody according to any of the first to the sixth aspects described above, or the monoclonal antibody or the antigen-binding fragment according to any of the seventh to the tenth aspects described above in the preparation of a medicament or a kit or an antibody conjugate.

### BRIEF DESCRIPTION OF THE DRAWINGS

FIG. 1 shows a schematic of the structure of a bispecific antibody provided according to an example of the present invention.
FIG. 2 shows a schematic of the structure of a bispecific antibody provided according to an example of the present invention.
FIG. 3 shows a schematic of the structure of a bispecific antibody provided according to an example of the present invention.
FIG. 4 shows a schematic of the structure of a bispecific antibody provided according to an example of the present invention.
FIG. 5 shows the results of measuring the binding activity of antibodies to GARP-TGF-β complex using FACS, provided according to an example of the present invention.
FIG. 6 shows the binding activity results of antibodies to TGF-β, provided according to an example of the present invention.
FIG. 7 is a graph showing the neutralizing activity results of antibodies against TGF-β, provided according to an example of the present invention.
FIG. 8 is a graph showing the pharmacodynamic results of antibodies in an animal model, provided according to an example of the present invention.

### DETAILED DESCRIPTION

The technical solutions of the present invention are described in detail below with reference to specific examples. Meanwhile, to facilitate understanding by those skilled in the art, some terms of the present invention are explained and described. It should be noted that these explanations and descriptions are merely used to facilitate understanding by those skilled in the art and should not be construed as limiting the protection scope of the present invention.

The term "bispecific antibody" comprises antigen-binding moieties that specifically bind to epitopes of at least two different biomolecules, and for the sake of distinction, the antigen-binding moieties are referred to as "first antigen-binding moiety" and "second antigen-binding moiety". The "first antigen-binding moiety" and "second antigen-binding moiety" are also explained and described below. The order in which the bispecific antibodies listed bind to the antigens is arbitrary unless otherwise indicated. Certainly, when specifically binding to a different biomolecule, a bispecific antibody may bind to more than one epitope of that particular biomolecule.

The term "antibody" is used herein in its broadest sense to refer to a protein or polypeptide that comprises an antigen-binding site, and to encompass natural and artificial antibodies of various structures, including but not limited to intact antibody forms or antigen-binding fragments of antibodies.

"Intact antibody" or "complete antibody" or the like is used herein to present the same meaning-a protein that comprises at least two heavy chains (H) and two light chains (L) that are linked to each other by disulfide bonds. Each heavy chain consists of a heavy chain variable region (abbreviated as VH) and a heavy chain constant region (abbreviated as CH). The heavy chain constant region comprises a first heavy chain constant domain (CH1), a second heavy chain constant domain (CH2), and a third heavy chain constant domain (CH3). Each light chain consists of a light chain variable region (abbreviated as VL) and a light chain constant region. The light chain constant region is also known as the light chain constant domain (CL). The VH and VL regions can be further divided into complementarity-determining regions (also known as hypervariable regions, abbreviated as CDRs) with conserved framework regions (FRs) interposed therebetween. Each VH and VL comprises three CDRs and four FRs, which are arranged from the amino-terminus (N-terminus) to the carboxy-terminus (C-terminus) as follows: FR1, CDR1, FR2, CDR2, FR3, CDR3, and FR4. Starting from the amino terminus of the heavy chain amino acid sequence, the CDRs of the heavy chain variable region are referred to as HCDR1, HCDR2, and HCDR3. Starting from the amino terminus of the light chain amino acid sequence, the CDRs of the light chain variable region are referred to as LCDR1, LCDR2, and LCDR3.

"Antigen-binding fragment" or "antigen-binding moiety" (e.g., the first antigen-binding moiety or the second antigen-binding moiety) refers to comprising a portion of the full-length antibody, typically a variable domain or region that binds to the antigen. The term "variable region" or "variable domain" refers to a domain that is involved in the binding of an antigen to the heavy or light chain of an antibody. As mentioned above, the variable regions of the heavy and light chains of an intact antibody generally have similar results, and each domain comprises four conserved framework regions and three hypervariable regions. Examples of antigen-binding fragments or antigen-binding moieties may be Fab, Fab', F(ab')2, bispecific Fab' and Fv fragments, linear antibodies, single-chain antibodies, single-domain antibodies, etc. Digestion of an intact antibody with papain produces two identical antigen-binding fragments, which are referred to as Fab fragments and each comprises a heavy chain variable region and a light chain variable region, as well as a light chain constant domain and a first heavy chain constant domain (CH1). Fab' fragments differ from Fab fragments by the addition of a few residues at the carboxy-terminus of the first heavy chain constant domain CH1, including one or more cysteines from the antibody hinge region. Digestion of an intact antibody with pepsin produces a F(ab')2 fragment. The F(ab')2 fragment has two antigen-binding F(ab) moieties that are linked together by a disulfide bond. The F(ab')2 fragment is a bivalent antibody. A single-chain antibody is a fusion protein formed by linking an antibody heavy chain variable region and an antibody light chain variable region through a flexible short peptide consisting of about 10-25 amino acids. A single-domain antibody is an antibody fragment consisting of a variable region of a single monomer. Single-domain antibodies are often referred to as nanobodies as they are typically derived from the heavy chain variable regions of antibodies from animals of the family Camelidae or the family Carcharhinidae.

For example, nanobodies comprise only heavy chain CDRs; they are the smallest antigen-binding fragments with full function. A nanobody comprises three CDRs (CDR1-CDR3) and four framework regions (FRs, FR1-FR4). The framework region FR1, the framework region FR2, the framework region FR3, and the framework region FR4 are separated by the complementarity-determining region CDR1, the complementarity-determining region CDR2, and the complementarity-determining region CDR3.

The term "and/or", when used to connect two or more selectable items, should be understood to mean any of the selectable items or any two or more of the selectable items.

The term "comprise" or "include" means including the stated elements or steps but not excluding other elements or steps. Certainly, "comprise" or "include" also encompasses instances consisting of the stated elements or steps, unless otherwise stated. For example, when referring to an antibody variable region comprising a particular sequence, it is also intended to encompass an antibody variable region that consists of that particular sequence.

The "affinity" or "binding affinity" mentioned herein is to be understood in the general sense of the art and is used to reflect the strength and/or stability between an antigen and a binding site on an antibody or antigen-binding fragment.

"Specifically binding" or "specifically binding to", "binding to", or "specifically targeting" a particular antigen or epitope, or being "specific for" or "capable of binding to" a particular antigen or epitope means being distinguished from non-specific interactions; such specific binding can be measured through some commonly used methods in the art. The ability of an antibody to bind to an antigen can be measured through the enzyme-linked immunosorbent assay (ELISA) or other techniques familiar to those skilled in the art. For example, antigen-carrying cells can be detected by flow cytometry, and the competitive binding between the test antibody and the labeled antibody can be detected by measuring the positive rate of cells. Since the spatial structures of the antigens on the cell surface are more similar to the forms existing *in vivo,* the real situation can be better reflected through that method. According to a specific embodiment of the present invention, the antibody provided has an EC50 value of ≤100 nM, ≤50 nM, ≤20 nM, ≤10 nM, ≤5 nM, ≤1 nM, ≤0.5 nM, ≤0.1 nM, or ≤0.05 nM. In addition, the binding activity of an antibody to an antigen can also be measured through surface plasmon resonance (SPR) technology or bio-layer interferometry (BLI) technology.

Whether what is provided herein is a monoclonal antibody or an antigen-binding fragment, or a bispecific antibody, or a polynucleotide, it is typically isolatable or recombinant. "Isolatable" means being capable of being identified and isolated and/or collected from a cell or cell culture where a polypeptide or protein is expressed. Typically, an isolated polypeptide is prepared through at least one purification step. "Isolated antibody" refers to being substantially free of having different antigens. "Recombinant" means that the antibody can be produced in an exogenous host cell using genetic recombination technology.

"Humanized" antibodies generally refer to antibodies consisting of antigen-binding moieties derived from non-human species and some structures and sequences based on human immunoglobulin molecules. For example, in a humanized antibody, the entire antibody, except for the CDRs, is encoded by a human-derived polynucleotide; it retains the binding activity to the antigen while having reduced immunogenicity.

The CDR sequences of the first antigen-binding moiety and the second antigen-binding moiety shown herein are obtained by analysis according to existing definition schemes; for example, the CDR sequences of the first antigen-binding moiety and the CDR sequences of the second antigen-binding moiety are obtained according to the IMGT definition scheme. These sequences can also be obtained through Kabat (e.g., see U.S. Dept. of Health and Human Servies, "Sequences of Proteins of Immunological Interest" (1983)), Chothia (e.g., see J. Mol. Biol. 196:901-917(1987)), or other definition schemes. It will be appreciated by those skilled in the art that different CDRs resulting from differences in the definition methods are all within the protection scope of the present invention.

### Bispecific Antibody

The present invention provides a bispecific antibody, comprising a first antigen-binding moiety and a second antigen-binding moiety, wherein the first antigen-binding moiety and the second antigen-binding moiety target different target antigens. Herein, the first antigen-binding moiety is capable of specifically targeting one, two, or three of TGFβ, GARP, and GARP-TGF-β complex; the second antigen-binding moiety specifically targets PD-L1. Whether the first antigen-binding moiety specifically targets TGFβ, or GARP, or GARP-TGFβ complex, or any two or three of the foregoing, it can directly or indirectly achieve regulation of TGF-β, activate immune responses to cancer cells, and inhibit disease progression. It should be noted that the terms "first" and "second", as used herein, are used for descriptive purposes only and should not be construed as indicating or implying relative importance or implicitly indicating the number of indicated technical features, nor should they be used to indicate an order of appearance. The terms "first antigen-binding moiety" and "second antigen-binding moiety" mean moieties that are capable of binding to different target antigens; they may be intact antibodies or part of an intact antibody, e.g., heavy chain variable regions, Fab, Fab', F(ab')₂, single-chain antibodies (ScFvs), single-domain antibodies (sdAbs), or the like.

The present invention provides a bispecific antibody, comprising a first antigen-binding moiety that specifically binds to TGF-β, GARP, and/or GARP-TGF-β complex, and a second antigen-binding moiety that specifically binds to PD-L1; further comprising a first heavy chain constant domain CH1 and a light chain constant domain CL, wherein the first antigen-binding moiety is covalently linked to the first heavy chain constant domain CH1 and the light chain constant domain CL; and further comprising an Fc region, wherein the Fc region is linked to the first heavy chain constant domain CH1 by a hinge region, and the second antigen-binding moiety is covalently linked to the Fc region and/or the light chain constant domain CL by a linker. Sequences of the formed IgG-type structure, except for the heavy chain variable region and the light chain variable region mentioned, can be natural sequences derived from mammals (e.g., human-derived natural sequences). In one specific embodiment, a sequence formed from the first heavy chain constant domain, the second heavy chain constant domain, and the third heavy chain constant domain is set forth in SEQ ID NO: 92.







 (SEQ ID NO: 92)

In one specific embodiment, the light chain constant domain sequence is set forth in SEQ ID NO: 93.



 (SEQ ID NO: 93)

The Fc region sequence of the bispecific antibody provided is not limited; it may be a naturally derived Fc region, e.g., a human-derived Fc region, or may be a modified Fc region. The modified Fc region may be a mutated Fc region; for example, certain sites in the Fc region may be mutated to adjust the ADCC effect function of the antibody.

The bispecific antibody provided has at least one of the following properties: (a) specifically binding to PD-L1; (b) specifically binding to glycoprotein A repetitions predominant (GARP); (c) specifically binding to GARP-TGF-β complex; (d) specifically binding to TGF-β; (e) having an activity-regulating function on immune cells, including but not limited to having inhibitory activity against the immunosuppressive function of regulatory T cells, etc.; and (f) having anti-tumor activity.

The bispecific antibody provided by the present invention shows simultaneously activity of specifically binding to TGF-β, GARP, and/or GARP-TGF-β complex, and activity of specifically binding to PD-L1. Through assay methods such as TGF-β reporter gene experiments, Treg release TGF-β neutralization experiments, etc., the bispecific antibody has been determined to be capable of both binding to GARP-TGF-β1 complex and capturing differential molecules of TGF-β1 released into the microenvironment. In addition, the bispecific antibody provided is capable of specifically binding to PD-L1; by simultaneously blocking the PD-1/PD-L1 and TGF-β signaling pathways, the bispecific antibody provided can eliminate immunosuppression and prevent possible complementary effects between the PD-L1 (PD-1) axis and TGF-β axis pathways, greatly restoring the body's immunity to achieve an enhanced killing effect.

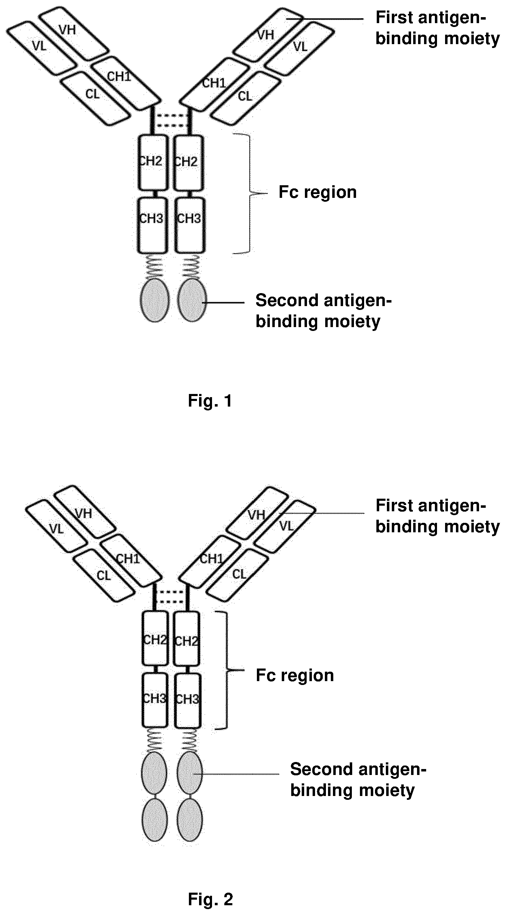

In at least some embodiments, the second antigen-binding moiety is linked to each of the carboxy-termini (C-termini) of the Fc region by a linker, as shown in FIG. 1 and FIG. 2. It should be noted that disulfide bonds are not specifically depicted in the given schematics. FIG. 1 shows that each of the carboxy-termini (C-termini) of the Fc region is linked to one second antigen-binding moiety. The second antigen-binding moiety may be configured as a Trap structure, a nanobody, or a single-chain antibody, if desired, or may be another fusion protein capable of binding to PD-L1. According to a specific embodiment, the second antigen-binding moiety may be an anti-PD-L1 nanobody. When the C-termini of the Fc region are linked to nanobodies, they may be linked to identical nanobodies or different nanobodies, respectively. Moreover, it is also possible to link only one of the C-termini of the Fc to a nanobody, if desired. FIG. 2 shows that the C-termini of the Fc region are linked to two second antigen-binding moieties, respectively. According to a specific embodiment, the C-termini of the Fc are linked to two PD-L1 nanobodies, respectively. Likewise, the C-termini of the Fc region may be linked to identical nanobodies or different nanobodies, respectively. Moreover, the number of nanobodies to which each of the C-termini of the Fc region is linked may be the same (e.g., two or more) or different (e.g., two or more). Taking any of the carboxy-termini of the Fc region as an example, the two or more nanobodies to which it is linked may be identical or different. The anti-PD-L1 nanobodies may be published or commercially available nanobodies, or may be obtained by screening an antibody library. For example, Chinese Patent Application No. 202110903293.4 describes that an anti-PD-L1 nanobody library was obtained by an alpaca immunization method, and that screening and identification were performed to obtain nanobodies with good affinities, specific binding, and tumor-killing effects. The international patent application No. PCT/CN2022/110423 (priority application No. 202110903293.4) is incorporated herein by reference in its entirety or in part as needed. According to a specific embodiment, each of the C-termini of the Fc may be linked to one single-chain antibody. The PD-L1 single-chain antibody may be a published or commercially available single-chain antibody. The single-chain antibodies to which the C-termini of the Fc are linked may be identical or different; it is also possible to link only one of the C-termini of the Fc to a single-chain antibody.

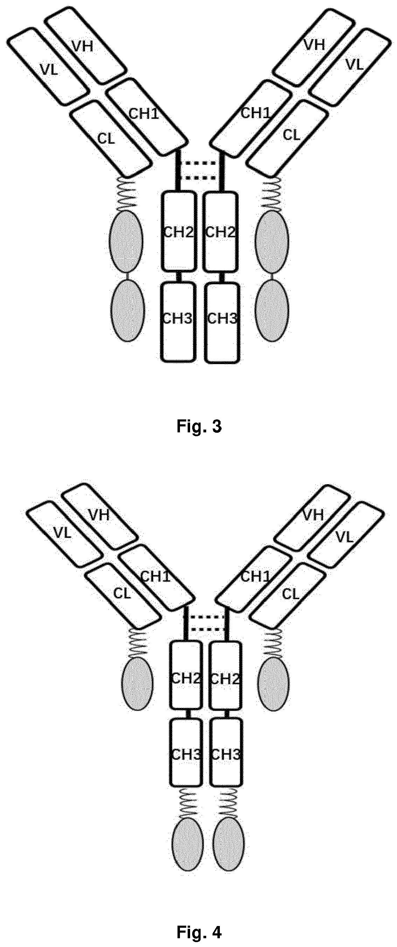

In at least some embodiments, the second antigen-binding moiety is linked to the carboxy-terminus (C-terminus) of the light chain constant domain by a linker. In at least some specific embodiments, the second antigen-binding moiety is linked to each of the carboxy-termini of the light chain constant domains by a linker, as shown in FIG. 3. The second antigen-binding moiety may be a single-chain antibody, a single-domain antibody, a fusion protein in another form, or the like, if desired. The second antigen-binding moieties to which the carboxy-termini of the light chain constant domains are linked may be identical or different, or only one of the carboxy-termini of the light chain constant domains is linked to a second antigen-binding moiety. In some specific embodiments, each of the carboxy-termini (C-termini) of the light chain constant domains is linked to two anti-PD-L1 nanobodies. Likewise, each of the C-termini of the light chain constant domains may be linked to one anti-PD-L1 nanobody or two or more anti-PD-L1 nanobodies or the like, if desired. Taking any of the carboxy-termini of the light chain constant domains as an example, the number of anti-PD-L1 nanobodies to which it is linked may be the same or different.

In at least some embodiments, the second antigen-binding moiety is linked to each of the C-termini of the Fc region by a first linker and to each of the C-termini of the light chain constant domains by a second linker, as shown in FIG. 4. Likewise, the second antigen-binding moieties linked to the C-termini of the Fc region or the second antigen-binding moieties linked to the light chain constant domains may be identical or different. The second antigen-binding moieties may be Trap structures, single-chain antibodies, single-domain antibodies, or fusion proteins in other forms, if desired. The first linker and the second linker may be identical or different.

Herein, useful linkers are those that are commonly used in the art and may be some oligopeptides or polypeptides. These oligopeptides or polypeptides may be any amino acid sequences that are capable of providing flexibility. Linkers include, but are not limited to, the group consisting of GS, SG, GGS, GSG, SGG, GGG, GGGS, SGGG, GGGGS, GGGGGSGS, GGGGSGS, GGGGSGGS, GGGGSGGGGSGGGGS, GGGGSGGGGS, GGGGSGGGGSGGGGSGGGGS, GGGGSGGGGSGGGGSGGGGSGGGGS, GGGGSGGGGSGGGGSGGGGSGGGSGGGS, etc.

By binding to TGF-β, GARP, or GAR-P-TGFβ complex and to PD-L1, the bispecific antibody provided activates immune responses to cancer cells and inhibits disease progression.

The present invention provides a bispecific antibody, comprising a first antigen-binding moiety and a second antigen-binding moiety, wherein the first antigen-binding moiety comprises: the HCDR1, HCDR2, and HCDR3 sequences of a heavy chain variable region set forth in SEQ ID NO: 36 or 37 or 38 or 39 or 40 or 41 or 42 or 43 or 44 or 45 or 46 or 47 and the LCDR1, LCDR2, and LCDR3 sequences of a light chain variable region set forth in SEQ ID NO: 48 or 49 or 50 or 51 or 52 or 53 or 54 or 55 or 56 or 57 or 58 or 59; the second antigen-binding moiety is capable of binding to PD-L1. The CDR sequences of the heavy chain variable region and light chain variable region sequences are somewhat different in that the analysis methods for antibody sequences are different, e.g., the classical Kabat CDR definition scheme, IMGT definition scheme, Chothia definition scheme, etc. mentioned above. Different CDR sequences obtained by analysis are all within the protection scope of the present invention.

The present invention further provides a bispecific antibody, comprising a first antigen-binding moiety and a second antigen-binding moiety, wherein the second antigen-binding moiety is capable of binding to PD-L1; the first antigen-binding moiety comprises a heavy chain variable region, and the heavy chain variable region comprises HCDR sequences set forth in SEQ ID NOs: 1, 2, and 3, or sequences that have one or two amino acid substitutions, deletions, or additions compared to the HCDR sequences set forth in SEQ ID NOs: 1, 2, and 3; or comprises HCDR sequences set forth in SEQ ID NOs: 4, 5, and 6, or sequences that have one or two amino acid substitutions, deletions, or additions compared to the HCDR sequences set forth in SEQ ID NOs: 4, 5, and 6; or comprises HCDR sequences set forth in SEQ ID NOs: 7, 8, and 9, or sequences that have one or two amino acid substitutions, deletions, or additions compared to the HCDR sequences set forth in SEQ ID NOs: 7, 8, and 9; or comprises HCDR sequences set forth in SEQ ID NOs: 10, 11, and 12, or sequences that have one or two amino acid substitutions, deletions, or additions compared to the HCDR sequences set forth in SEQ ID NOs: 10, 11, and 12; or comprises HCDR sequences set forth in SEQ ID NOs: 13, 14, and 15, or sequences that have one or two amino acid substitutions, deletions, or additions compared to the HCDR sequences set forth in SEQ ID NOs: 13, 14, and 15; or comprises HCDR sequences set forth in SEQ ID NOs: 16, 17, and 18, or sequences that have one or two amino acid substitutions, deletions, or additions compared to the HCDR sequences set forth in SEQ ID NOs: 16, 17, and 18.

In some embodiments, the first antigen-binding moiety may further comprise a light chain variable region, and the light chain variable region comprises LCDR sequences set forth in SEQ ID NOs: 19, 20, and 21, or sequences that have one or two amino acid substitutions, deletions, or additions compared to the LCDR sequences set forth in SEQ ID NOs: 19, 20, and 21; or comprises LCDR sequences set forth in SEQ ID NOs: 22, 23, and 24, or sequences that have one or two amino acid substitutions, deletions, or additions compared to the LCDR sequences set forth in SEQ ID NOs: 22, 23, and 24; or comprises LCDR sequences set forth in SEQ ID NOs: 25, 20, and 26, or sequences that have one or two amino acid substitutions, deletions, or additions compared to the LCDR sequences set forth in SEQ ID NOs: 25, 20, and 26; or comprises LCDR sequences set forth in SEQ ID NOs: 27, 28, and 29, or sequences that have one or two amino acid substitutions, deletions, or additions compared to the LCDR sequences set forth in SEQ ID NOs: 27, 28, and 29; or comprises LCDR sequences set forth in SEQ ID NOs: 30, 31, and 32, or sequences that have one or two amino acid substitutions, deletions, or additions compared to the LCDR sequences set forth in SEQ ID NOs: 30, 31, and 32; or comprises LCDR sequences set forth in SEQ ID NOs: 33, 34, and 35, or sequences that have one or two amino acid substitutions, deletions, or additions compared to the LCDR sequences set forth in SEQ ID NOs: 33, 34, and 35.

The sequences are shown below. The first antigen-binding moiety or the second antigen-binding moiety of the bispecific antibody mentioned may be an intact antibody or only a fragment thereof if desired, so long as it is capable of targeting a different target antigen. The first antigen-binding moiety or the second antigen-binding moiety may be simply a heavy chain variable region, Fab, Fab', F(ab')₂, a single-chain antibody (ScFv), a single-domain antibody (sdAb), or the like.

| Sequence No. (SEQ ID NO:) | HCDR1 | Sequence No. (SEQ ID NO:) | HCDR2 | Sequence No. (SEQ ID NO:) | HCDR3 |
|---|---|---|---|---|---|
| 1 | GYIFTSYW | 2 | IAPNSGGA | 3 | ARGNYGMDY |
| 4 | GYTFTDYN | 5 | INPYSGGT | 6 | ARVSKGSGDY |
| 7 | GYTFSAYT | 8 | INPSNDFA | 9 | ARVDYYGGSYRWYLDV |
| 10 | GYTFTSYW | 11 | INPSNGDT | 12 | ARATYGSYGYFDV |
| 13 | GFSLTTYG | 14 | IWGDGTT | 15 | ASYYFAMHY |
| 16 | GFTFSDYY | 17 | ISNGGGRT | 18 | ARQKTNYGNYEEDYYAMGY |

| Sequence No. (SEQ ID NO:) | LCDR1 | Sequence No. (SEQ ID NO:) | LCDR2 | Sequence No. (SEQ ID NO:) | LCDR3 |
|---|---|---|---|---|---|
| 19 | QDISNY | 20 | YTS | 21 | QQVNTLPFT |
| 22 | QEISGY | 23 | AAS | 24 | LQYASYPTCT |
| 25 | QSISNN | 20 | YTS | 26 | QQSNSWPIFT |
| 27 | ESVTIIGTNL | 28 | HAS | 29 | LQSRQIPRT |
| 30 | QSLLNSRTQKNY | 31 | WAS | 32 | KQSYNLWT |
| 33 | KSVSTSGYSY | 34 | LAS | 35 | QHSRELPLTFGAGTK |

The first antigen-binding moiety of the bispecific antibody provided is capable of binding to GARP, TGFβ, or GARP-TGFβ complex, or is capable of specifically binding to any two or three of the foregoing. Meanwhile, it is capable of binding to PD-L1, blocking the PD-1/PD-L1 pathway, and enhancing the killing effect of immune cells on tumor cells.

In some specific embodiments, the bispecific antibody provided has HCDR sequences set forth in SEQ ID NOs: 1, 2, and 3 and LCDR sequences set forth in SEQ ID NOs: 19, 20, and 21. In some specific embodiments, the bispecific antibody provided has HCDR sequences set forth in SEQ ID NOs: 4, 5, and 6 and LCDR sequences set forth in SEQ ID NOs: 22, 23, and 24. In some embodiments, the bispecific antibody provided has HCDR sequences set forth in SEQ ID NOs: 7, 8, and 9 and LCDR sequences set forth in SEQ ID NOs: 25, 20, and 26. In some embodiments, the bispecific antibody provided has HCDR sequences set forth in SEQ ID NOs: 10, 11, and 12 and LCDR sequences set forth in SEQ ID NOs: 27, 28, and 29. In some specific embodiments, the bispecific antibody provided has HCDR sequences set forth in SEQ ID NOs: 13, 14, and 15 and LCDR sequences set forth in SEQ ID NOs: 30, 31, and 32. In some specific embodiments, the bispecific antibody provided has HCDR sequences set forth in SEQ ID NOs: 16, 17, and 18 and LCDR sequences set forth in SEQ ID NOs: 33, 34, and 35. The HCDR sequences and LCDR sequences shown serve as the first antigen-binding moiety of the bispecific antibody and specifically bind to the target antigen.

In some embodiments, the bispecific antibody provided comprises at least one of the following: a heavy chain variable region that has at least 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence set forth in SEQ ID NO: 36 or 37 or 38 or 39 or 40 or 41 or 42 or 43 or 44 or 45 or 46 or 47, and a light chain variable region that has at least 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence set forth in SEQ ID NO: 48 or 49 or 50 or 51 or 52 or 53 or 54 or 55 or 56 or 57 or 58 or 59. SEQ ID NOs: 42, 43, 44, 45, 46, and 47 are humanized heavy chain variable region sequences. SEQ ID NOs: 54, 55, 56, 57, 58, and 59 are humanized light chain variable region sequences. The heavy chain variable region and light chain variable region sequences shown serve as the first antigen-binding moiety of the bispecific antibody and specifically bind to the target antigen. The sequences of the light chain variable region and the heavy chain variable region of the first antigen-binding moiety are less immunogenic after humanization and retain the binding activity to the target antigen (e.g., GARP, TGFβ, and/or GARP-TGFβ complex). The humanized antibody sequence may be obtained through methods commonly used in the art (e.g., complementarity-determining region grafting). In some embodiments, the humanized antibody sequence provided retains the CDR sequences and has human framework region sequences in place of the framework region sequences. Human framework region sequences may be obtained from published human sequences. For example, human FR templates with the greatest homology are found through database alignment and computer homology modeling; whether the FRs need to be back-mutated and key residues for the back mutation are determined by comprehensive consideration; and a humanized antibody with high affinity is obtained. These sequences are usually set forth in commonly used databases, such as the PDB protein structure database, IMGT, Genebank, etc.

The "sequence identity" mentioned herein refers to the degree to which amino acid or nucleotide sequences are identical when compared one by one. The determination of sequence identity ratios can be accomplished in a variety of ways known in the art. For example, homology can be obtained by using publicly available software such as BLAST, ALIGN, BLAST-2, etc. In some specific embodiments, sequence identity results from conservative amino acid substitutions. "Conservative amino acid substitution" refers to the replacement of an amino acid residue with a different amino acid residue that has a side chain with similar physiochemical properties. For example, conservative amino acid substitutions may be made between amino acid residues that have hydrophobic side chains (e.g., Met, Ala, Val, Leu, and Ile), between residues that have neutral hydrophilic side chains (e.g., Cys, Ser, Thr, Asn, and Gln), between residues that have acidic side chains (e.g., Asp and Glu), between amino acids that have basic side chains (e.g., His, Lys, and Arg), or between residues that have aromatic side chains (e.g., Trp, Tyr, and Phe). As is known in the art, conservative amino acid substitutions typically do not cause significant changes in the conformational structure of a protein; thus, the biological activity of the protein can be preserved. The conservative amino acid substitutions mentioned may be 1 conservative amino acid substitution, 2 conservative amino acid substitutions, 3 conservative amino acid substitutions, 4 conservative amino acid substitutions, 5 conservative amino acid substitutions, 6 conservative amino acid substitutions, 7 conservative amino acid substitutions, 8 conservative amino acid substitutions, 9 conservative amino acid substitutions, 10 conservative amino acid substitutions, or the like. The names of amino acids used herein are indicated by one-letter or three-letter codes as is standard in the art.

The sequences are shown below:

| Functional region | Sequence No. (SEQ ID NO:) | Amino acid sequence |
|---|---|---|
| VH | 36 | QVQLQQPGPELVKPGASVKLTCKASGYIFTSYWMHWVKRGPGRGL EWIGRIAPNSGGAHYHEKFKTKVTLTVDKPSSTAYMEFSSLTSEDSA VYYCARGNYGMDYWGQGTAVTVSS |
| VH | 37 | EVQLQQSGPELVKPGASVKIPCKTSGYTFTDYNMNWVKQSHGKSL EWIGDINPYSGGTIYNQKFKGKAILTVDKSSSTAYMELRSLTSEDTGI YYCARVSKGSGDYWGQGTTLTVSS |
| VH | 38 | QVHLQQSGAALARPGASVKMSCKASGYTFSAYTIHWVKQRPGQG LEWIGYINPSNDFAKYIQKFKDKATLTADKSSSTAYMQLNSLTSED SAIYYCARVDYYGGSYRWYLDVWGAGTTVTVSS |
| VH | 39 | QVQLQQPGTELVKPGASVKLSCKASGYTFTSYWMHWVKQRPGQG LEWIGNINPSNGDTNYNEKFKSKATLTVDKSSSTAYMQLSSLTSDDS AVYYCARATYGSYGYFDVWGTGTAVTVSS |
| VH | 40 | QVQLKESGPGLVAPSQSLSITCTVSGFSLTTYGVTWVRQPPGKGLQ WLGLIWGDGTTDYHSTFISRLSISKDKSKNQVFFKLNSLQTDDTATY YCASYYFAMHYWGQGTSVTVSS |
| VH | 41 | EVKLVESGGGLVQPGGSLKLSCATSGFTFSDYYMYWVRQTPEKRL EWVAYISNGGGRTYYPDTVKGRFTISRDNAKNTLYLQMSRLKSEDR AIYYCARQKTNYGNYEEDYYAMGYWGQGTSVTVSS |
| VH-hu | 42 | QVQLVQSGAEVKKPGSSVKVSCKASGYIFTSYWMHWVKQAPGQG LEWIGRIAPNSGGAHYHEKFKTKVTITADKSTSTAYMEFSSLRSEDT AVYYCARGNYGMDYWGQGTAVTVSS |
| VH-hu | 43 | QVQLVQSGAEVKKPGASVKVSCKTSGYTFTDYNMNWVKQAPGQ GLEWIGDINPYSGGTIYNQKFKGRATLTTDTSTSTAYMELRSLRSDD TAVYYCARVSKGSGDYWGQGTLVTVSS |
| VH-hu | 44 | QVQLVQSGAEVKRPGASVKVSCKASGYTFSAYTIHWVKQRPGQGL EWIGYINPSNDFAKYIQKFKDKVTITADKSTSTAYMELNSLRSEDTA VYYCARVDYYGGSYRWYLDVWGQGTTVTVSS |
| VH-hu | 45 | QVQLVQSGAEVKKPGASVKVSCKASGYTFTSYWMHWVKQAPGQ GLEWIGNINPSNGDTNYNEKFKSKATLTVDKSTSTAYMELRSLRSD DTAVYYCARATYGSYGYFDVWGQGTLVTVSS |
| VH-hu | 46 | QVQLQESGPGLVKPSGTLSLTCAVSGFSLTTYGVTWVRQPPGKGLE WIGLIWGDGTTDYHSTFISRLTISKDKSKNQVSLKLSSVTAADTAVY YCASYYFAMHYWGQGTLVTVSS |
| VH-hu | 47 | EVQLVESGGGLVQPGGSLRLSCATSGFTFSDYYMYWVRQTPEKRLE WVAYISNGGGRTYYPDTVKGRFTISRDNAKNSLYLQMSRLRAEDTA VYYCARQKTNYGNYEEDYYAMGYWGQGTSVTVSS |
| VL | 48 | DIQMTQTTSSLSASLGDRVTISCRASQDISNYLNWYQQKPDGTVEL LIYYTSTLHSGVPSRFSGSGSGTDYSLTISNLEQEDIATYFCQQVNTL PFTFGSGTKLEII |
| VL | 49 | DIQMTQSPSSLSASLGERVSLTCRASQEISGYLSWLQQKPDGTIKRLI YAASTLDSGVPKRFSGSRSGSDYSLTISSLESEDFADYYCLQYASYPT CTFGGGTKLEIK |
| VL | 50 | DIVLTQSPATLSVTPGDSVSLSCRASQSISNNLHWYQQKSHESPRLLI KYTSQSISGIPSRFSGSGSGTDFTLSINSVETEDFGMYFCQQSNSWPI FTFGSGTKLEIK |
| VL | 51 | DIVLTQSPASLAMSLGKRATISCRASESVTIIGTNLIQWFQQKPGQPP KLLIYHASNLETGVPARFSGSGSRTDFTLTIDPVEEDDVAIYYCLQSR QIPRTFGGGTKLEIK |
| VL | 52 | DIVMSQSPSSLAVSAGEKVTMNCKSSQSLLNSRTQKNYLAWYQQK PGQSPKLLIYWASTRESGVPDRFTGSGSGTDFTLTIISVQAEDLAVYY CKQSYNLWTFGGGTKLEIK |
| VL | 53 | DIVLTQSPASLAVSLGQRATISCRASKSVSTSGYSYIHWYQQKPGQP PKLLIYLASNLESGVPARFSGSGSGTDFTLNIHPVEEEDAATYYCQH SRELPLTFGAGTKLDLK |
| VL-hu | 54 | DIQMTQSPSSLSASVGDRVTITCRASQDISNYLNWYQQKPGGAPEL LIYYTSTLHSGVPSRFSGSGSGTDYTLTISSLQPEDFATYFCQQVNTL PFTFGSGTKLEIK |
| VL-hu | 55 | DIQMTQSPSSLSASVGDRVTITCRASQEISGYLSWLQQKPGGAPKRL IYAASTLDSGVPSRFSGSRSGTDYTLTISSLQPEDFATYYCLQYASYP TCTFGGGTKVEIK |
| VL-hu | 56 | DIVLTQSPATLSLSPGERATLSCRASQSISNNLHWYQQKPGEAPRLLI KYTSQSISGIPARFSGSGSGTDFTLTISSLEPEDFAVYFCQQSNSWPIF TFGQGTKLEIK |
| VL-hu | 57 | DIVLTQSPDSLAVSLGERATINCRASESVTIIGTNLIQWFQQKPGQPP KLLIYHASNLETGVPDRFSGSGSRTDFTLTISSLQAEDVAVYYCLQSR QIPRTFGGGTKVEIK |
| VL-hu | 58 | DIVMTQSPDSLAVSLGERATINCKSSQSLLNSRTQKNYLAWYQQKP GQSPKLLIYWASTRESGVPDRFSGSGSGTDFTLTISSLQAEDVAVYY CKQSYNLWTFGGGTKVEIK |
| VL-hu | 59 | DIVLTQSPASLAVSLGQRATITCRASKSVSTSGYSYIHWYQQKPGQP PKLLIYLASNLESGVPARFSGSGSGTDFTLTINPVEEEDAATYYCQHS RELPLTFGAGTKLEIK |

In some specific embodiments, the first antigen-binding moiety comprises a heavy chain variable region set forth in SEQ ID NO: 36 and a light chain variable region set forth in SEQ ID NO: 48. In some specific embodiments, the first antigen-binding moiety comprises a heavy chain variable region set forth in SEQ ID NO: 37 and a light chain variable region set forth in SEQ ID NO: 49. In some specific embodiments, the first antigen-binding moiety comprises a heavy chain variable region set forth in SEQ ID NO: 38 and a light chain variable region set forth in SEQ ID NO: 50. In some specific embodiments, the first antigen-binding moiety comprises a heavy chain variable region set forth in SEQ ID NO: 39 and a light chain variable region set forth in SEQ ID NO: 51. In some specific embodiments, the first antigen-binding moiety comprises a heavy chain variable region set forth in SEQ ID NO: 40 and a light chain variable region set forth in SEQ ID NO: 52. In some specific embodiments, the first antigen-binding moiety comprises a heavy chain variable region set forth in SEQ ID NO: 41 and a light chain variable region set forth in SEQ ID NO: 53. In some specific embodiments, the first antigen-binding moiety comprises a heavy chain variable region set forth in SEQ ID NO: 42 and a light chain variable region set forth in SEQ ID NO: 54. In some specific embodiments, the first antigen-binding moiety comprises a heavy chain variable region set forth in SEQ ID NO: 43 and a light chain variable region set forth in SEQ ID NO: 55. In some specific embodiments, the first antigen-binding moiety comprises a heavy chain variable region set forth in SEQ ID NO: 44 and a light chain variable region set forth in SEQ ID NO: 56. In some specific embodiments, the first antigen-binding moiety comprises a heavy chain variable region set forth in SEQ ID NO: 45 and a light chain variable region set forth in SEQ ID NO: 57. In some specific embodiments, the first antigen-binding moiety comprises a heavy chain variable region set forth in SEQ ID NO: 46 and a light chain variable region set forth in SEQ ID NO: 58. In some specific embodiments, the first antigen-binding moiety comprises a heavy chain variable region set forth in SEQ ID NO: 47 and a light chain variable region set forth in SEQ ID NO: 59.

The second antigen-binding moiety is capable of specifically targeting PD-L1. As is mentioned above, the form of the second antigen-binding moiety is not limited so long as it is capable of specifically targeting PD-L1. For example, it may be an intact antibody or part of an intact antibody, including but not limited to a heavy chain variable region, Fab, Fab', F(ab')₂, a single-chain antibody, a nanobody, or the like. The second antigen-binding moiety may be obtained by screening an antibody library if desired, or may be a commercially available or published antibody sequence. In some specific embodiments, the second antigen-binding moiety is a single-domain antibody. In some preferred embodiments, the structure of the bispecific antibody formed is a structure in which an IgG-type antibody is linked to the second antigen-binding moiety by a linker, wherein the IgG-type antibody comprises the first antigen-binding moiety. The first antigen-binding moiety is combined with an Fc to form the IgG-type antibody. The second antigen-binding moiety is linked to the IgG-type antibody by a linker to form a bispecific antibody structure.

In some specific embodiments, the second antigen-binding moiety provided comprises a CDR1, a CDR2, and a CDR3, wherein the CDR1 comprises a sequence selected from the group consisting of: SEQ ID NOs: 60, 63, and 66, or sequences that have one or two amino acid substitutions, deletions, or additions compared to SEQ ID NOs: 60, 63, and 66; the CDR2 comprises a sequence selected from the group consisting of: SEQ ID NOs: 61, 64, and 67, or sequences that have one or two amino acid substitutions, deletions, or additions compared to SEQ ID NOs: 61, 64, and 67; and the CDR3 comprises a sequence selected from the group consisting of: SEQ ID NOs: 62, 65, 68, and 69, or sequences that have one, two, or three amino acid substitutions, deletions, or additions compared to SEQ ID NOs: 62, 65, 68, and 69.

In some specific embodiments, the second antigen-binding moiety has a CDR1 sequence set forth in SEQ ID NO: 60, a CDR2 sequence set forth in SEQ ID NO: 61, and a CDR3 sequence set forth in SEQ ID NO: 62. In some specific embodiments, the second antigen-binding moiety has a CDR1 sequence set forth in SEQ ID NO: 63, a CDR2 sequence set forth in SEQ ID NO: 64, and a CDR3 sequence set forth in SEQ ID NO: 65. In some specific embodiments, the second antigen-binding moiety has a CDR1 sequence set forth in SEQ ID NO: 66, a CDR2 sequence set forth in SEQ ID NO: 67, and a CDR3 sequence set forth in SEQ ID NO: 68. In some specific embodiments, the second antigen-binding moiety has a CDR1 sequence set forth in SEQ ID NO: 60, a CDR2 sequence set forth in SEQ ID NO: 61, and a CDR3 sequence set forth in SEQ ID NO: 69. The CDR1, CDR2, and CDR3 sequences shown may be combined with framework regions (FRs) to form a single-domian antibody. The framework region (FR) sequences are not particularly limited, and may be derived from mice or humans, or partially from mice and partially from humans.

The present invention further provides a bispecific antibody, wherein the bispecific antibody comprises a first antigen-binding moiety and a second antigen-binding moiety; the first antigen-binding moiety is capable of specifically binding to GARP, TGF-β, and/or GARP-TGF-β complex, and the second antigen-binding moiety is capable of specifically binding to PD-L1. In some specific embodiments, the second antigen-binding moiety is an anti-PD-L1 nanobody.

In some embodiments, the second antigen-binding moiety comprises a CDR1, a CDR2, and a CDR3, wherein the CDR1 comprises a sequence selected from the group consisting of: SEQ ID NOs: 60, 63, and 66, or sequences that have one or two amino acid substitutions, deletions, or additions compared to SEQ ID NOs: 60, 63, and 66; the CDR2 comprises a sequence selected from the group consisting of: SEQ ID NOs: 61, 64, and 67, or sequences that have one or two amino acid substitutions, deletions, or additions compared to SEQ ID NOs: 61, 64, and 67; and the CDR3 comprises a sequence selected from the group consisting of: SEQ ID NOs: 62, 65, 68, and 69, or sequences that have one, two, or three amino acid substitutions, deletions, or additions compared to SEQ ID NOs: 62, 65, 68, and 69. According to a specific embodiment of the present invention, the amino acid substitutions, deletions, or additions mentioned may result from conservative amino acid substitutions or from differences in characterizing CDRs using different definition schemes. It will be appreciated by those skilled in the art that CDR sequences obtained by characterization using different definition schemes are somewhat different, and these differences should also be within the protection scope of the present invention.

In some specific embodiments, the second antigen-binding moiety comprises: (1) a CDR1, which is the amino acid sequence set forth in SEQ ID NO: 60; a CDR2, which is the amino acid sequence set forth in SEQ ID NO: 61; and a CDR3, which is the amino acid sequence set forth in SEQ ID NO: 62; or (2) a CDR1, which is the amino acid sequence set forth in SEQ ID NO: 63; a CDR2, which is the amino acid sequence set forth in SEQ ID NO: 64; and a CDR3, which is the amino acid sequence set forth in SEQ ID NO: 65; or (3) a CDR1, which is the amino acid sequence set forth in SEQ ID NO: 66; a CDR2, which is the amino acid sequence set forth in SEQ ID NO: 67; and a CDR3, which is the amino acid sequence set forth in SEQ ID NO: 68; or (4) a CDR1, which is the amino acid sequence set forth in SEQ ID NO: 60; a CDR2, which is the amino acid sequence set forth in SEQ ID NO: 61; and a CDR3, which is the amino acid sequence set forth in SEQ ID NO: 69.

In at least some embodiments, the second antigen-binding moiety further comprises framework regions (FRs), including an FR1, an FR2, an FR3, and an FR4, wherein the FR1, FR2, FR3, and FR4 comprise the following amino acid sequences: (1) the FR1 comprises a sequence selected from SEQ ID NO: 70, SEQ ID NO: 74, SEQ ID NO: 77, and SEQ ID NO: 80, or a sequence that has one, two, or three conservative amino acid substitutions compared to SEQ ID NO: 70, 74, 77, or 80; (2) the FR2 comprises a sequence selected from SEQ ID NO: 71, SEQ ID NO: 75, and SEQ ID NO: 78, or a sequence that has one, two, or three conservative amino acid substitutions compared to SEQ ID NO: 71, 75, or 78; (3) the FR3 comprises a sequence selected from SEQ ID NO: 72, SEQ ID NO: 76, SEQ ID NO: 79, and SEQ ID NO: 81, or a sequence that has one, two, three, or four conservative amino acid substitutions compared to SEQ ID NO: 72, 76, 79, or 81; (4) the FR4 is selected from SEQ ID NO: 73, or a sequence that has one or two conservative amino acid substitutions compared to SEQ ID NO: 73.

In some specific embodiments, the framework regions include: an FR1, which is the amino acid sequence set forth in SEQ ID NO: 70; an FR2, which is the amino acid sequence set forth in SEQ ID NO: 71; an FR3, which is the amino acid sequence set forth in SEQ ID NO: 72; and an FR4, which is the amino acid sequence set forth in SEQ ID NO: 73. In some specific embodiments, the framework regions include: an FR1, which is the amino acid sequence set forth in SEQ ID NO: 74; an FR2, which is the amino acid sequence set forth in SEQ ID NO: 75; an FR3, which is the amino acid sequence set forth in SEQ ID NO: 76; and an FR4, which is the amino acid sequence set forth in SEQ ID NO: 73. In some specific embodiments, the framework regions include: an FR1, which is the amino acid sequence set forth in SEQ ID NO: 77; an FR2, which is the amino acid sequence set forth in SEQ ID NO: 78; an FR3, which is the amino acid sequence set forth in SEQ ID NO: 79; and an FR4, which is the amino acid sequence set forth in SEQ ID NO: 73. In some specific embodiments, the framework regions include: an FR1, which is the amino acid sequence set forth in SEQ ID NO: 80; an FR2, which is the amino acid sequence set forth in SEQ ID NO: 78; an FR3, which is the amino acid sequence set forth in SEQ ID NO: 79; and an FR4, which is the amino acid sequence set forth in SEQ ID NO: 73. In some specific embodiments, the framework regions include: an FR1, which is the amino acid sequence set forth in SEQ ID NO: 70; an FR2, which is the amino acid sequence set forth in SEQ ID NO: 71; an FR3, which is the amino acid sequence set forth in SEQ ID NO: 81; and an FR4, which is the amino acid sequence set forth in SEQ ID NO: 73.

In some embodiments, the second antigen-binding moiety is selected from the sequence set forth in SEQ ID NO: 82 or 83 or 84 or 85 or 86 or 87 or 88 or 89 or 90 or 91. In some embodiments, the second antigen-binding moiety has 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more sequence identity compared to the amino acid sequence set forth in SEQ ID NO: 82 or 83 or 84 or 85 or 86 or 87 or 88 or 89 or 90 or 91. The amino acid sequence set forth in SEQ ID NO: 88, 89, 90, 91, or 92 provided is a humanized antibody sequence. The humanized sequence provided has low immunogenicity and has a high affinity for the target protein.

| | Amino acid sequence |
|---|---|
| SEQ ID NO:60 | GRSFSSSG |
| SEQ ID NO:61 | INSSGGDT |
| SEQ ID NO:62 | AAKEGGGPSSIPAIYDY |
| SEQ ID NO:63 | GFTFSSYA |
| SEQ ID NO:64 | INTGSEIV |
| SEQ ID NO:65 | ATGLVSAEHDGI |
| SEQ ID NO:66 | GRDFLTYG |
| SEQ ID NO:67 | INWSGSMT |
| SEQ ID NO:68 | AARRGAVTYASSNEYEH |
| SEQ ID NO:69 | AAKEGGGPSSIPAIFDY |

| Sequence No. | Amino acid sequence |
|---|---|
| SEQ ID NO:70 | QVQLVESGGGLVQAGGSLRLSCAAS |
| SEQ ID NO:71 | MGWFRQAPGKDREFVAT |
| SEQ ID NO:72 | YYRDSVEGRFTTSRDNAKNTMSLQMNDLKPEDTAVYYC |
| SEQ ID NO:73 | WGQGTQVTVSS |
| SEQ ID NO:74 | QVQLVESGGGLVQPGGSLSLSCVAS |
| SEQ ID NO:75 | MRWVRQAPGKEPEWVAG |
| SEQ ID NO:76 | VYANSVRGRFAISRDNARDTLFLQMNSLKPEDTAVYYC |
| SEQ ID NO:77 | EVQLVESGGGSVQTGGSLRLSCVAS |
| SEQ ID NO:78 | MGWFRQAPGKEREFVAS |
| SEQ ID NO:79 | YYADSVKGRFTISRDNAKNTVYLQMNSLKPGDTAVYIC |
| SEQ ID NO:80 | EVQLVESGGGLVQAGDSLRLSCTAS |
| SEQ ID NO:81 | YYADSVEGRFTTSRDNAKNTMSLQMNDLKPEDTAVYYC |

| Sequence No. | Amino acid sequence |
|---|---|
| SEQ ID NO: 82 | QVQLVESGGGLVQAGGSLRLSCAASGRSFSSSGMGWFRQAPGKDREFVATINSS GGDTYYRDSVEGRFTTSRDNAKNTMSLQMNDLKPEDTAVYYCAAKEGGGPSSI PAIYDYWGQGTQVTVSS |
| SEQ ID NO: 83 | QVQLVESGGGLVQPGGSLSLSCVASGFTFSSYAMRWVRQAPGKEPEWVAGINTG SEIVVYANSVRGRFAISRDNARDTLFLQMNSLKPEDTAVYYCATGLVSAEHDGI WGQGTQVTVSS |
| SEQ ID NO: 84 | EVQLVESGGGSVQTGGSLRLSCVASGRDFLTYGMGWFRQAPGKEREFVASINW SGSMTYYADSVKGRFTISRDNAKNTVYLQMNSLKPGDTAVYICAARRGAVTYA SSNEYEHWGQGTQVTVSS |
| SEQ ID NO:85 | EVQLVESGGGLVQAGDSLRLSCTASGRDFLTYGMGWFRQAPGKEREFVASINW SGSMTYYADSVKGRFTISRDNAKNTVYLQMNSLKPGDTAVYICAARRGAVTYA SSNEYEHWGQGTQVTVSS |
| SEQ ID NO:86 | QVQLVESGGGLVQAGGSLRLSCAASGRSFSSSGMGWFRQAPGKDREFVATINSS GGDTYYADSVEGRFTTSRDNAKNTMSLQMNDLKPEDTAVYYCAAKEGGGPSSI PAIFDYWGQGTQVTVSS |
| SEQ ID NO:87 | QVQLVESGGGVVQPGGSLRLSCAASGRSFSSSGMGWFRQAPGKGREFVATINSS GGDTYYRDSVEGRFTTSRDNSKNTMYLQMNSLRAEDTAVYYCAAKEGGGPSSI PAIYDYWGQGTLVTVSS |
| SEQ ID NO:88 | QVQLVESGGGVVQPGGSLRLSCAASGFTFSSYAMRWVRQAPGKGLEWVAGINT GSEIVVYANSVRGRFTISRDNSRDTLYLQMNSLRAEDTAVYYCATGLVSAEHDGI WGQGTQVTVSS |
| SEQ ID NO: 89 | EVQLVESGGGLVQPGGSLRLSCAASGRDFLTYGMGWFRQAPGKGREFVASINW SGSMTYYADSVKGRFTISRDNAKNTVYLQMNSLKAEDTAVYICAARRGAVTYA SSNEYEHWGQGTLVTVSS |
| SEQ ID NO:90 | EVQLVESGGGLVQPGGSLRLSCTASGRDFLTYGMGWFRQAPGKELEFVASINWS GSMTYYADSVKGRFTISRDNAKNTVYLQMNSLRAEDTAVYICAARRGAVTYAS SNEYEHWGQGTQVTVSS |
| SEQ ID NO:91 | EVQLVESGGGLVQPGGSLRLSCAASGRDFLTYGMGWFRQAPGKGLEFVASINW SGSMTYYADSVKGRFTISRDNAKNSVYLQMNSLRAEDTAVYICAARRGAVTYA SSNEYEHWGQGTQVTVSS |

The present invention further provides a bispecific antibody, wherein the bispecific antibody comprises a first antigen-binding moiety and a second antigen-binding moiety; the first antigen-binding moiety is capable of binding to TGF-β, GARP, and/or GARP-TGF-β complex, and the second antigen-binding moiety is capable of binding to PD-L1.

In some specific embodiments, the first antigen-binding moiety comprises a heavy chain variable region and a light chain variable region, wherein the heavy chain variable region comprises HCDR sequences set forth in SEQ ID NOs: 1, 2, and 3, or comprises HCDR sequences set forth in SEQ ID NOs: 4, 5, and 6, or comprises HCDR sequences set forth in SEQ ID NOs: 7, 8, and 9, or comprises HCDR sequences set forth in SEQ ID NOs: 10, 11, and 12, or comprises HCDR sequences set forth in SEQ ID NOs: 13, 14, and 15, or comprises HCDR sequences set forth in SEQ ID NOs: 16, 17, and 18; the light chain variable region comprises LCDR sequences set forth in SEQ ID NOs: 19, 20, and 21, or comprises LCDR sequences set forth in SEQ ID NOs: 22, 23, and 24, or comprises LCDR sequences set forth in SEQ ID NOs: 25, 20, and 26, or comprises LCDR sequences set forth in SEQ ID NOs: 27, 28, and 29, or comprises LCDR sequences set forth in SEQ ID NOs: 30, 31, and 32, or comprises LCDR sequences set forth in SEQ ID NOs: 33, 34, and 35. The second antigen-binding moiety comprises a CDR1, a CDR2, and a CDR3, wherein the CDR1 comprises the sequence set forth in SEQ ID NO: 60 or 63 or 66, the CDR2 comprises the sequence set forth in SEQ ID NO: 61 or 64 or 67, and the CDR3 comprises the sequence set forth in SEQ ID NO: 62 or 65 or 68 or 69.

In some specific embodiments, the first antigen-binding moiety comprises a heavy chain variable region set forth in SEQ ID NO: 36 and a light chain variable region set forth in SEQ ID NO: 48; the second antigen-binding moiety comprises the sequence set forth in SEQ ID NO: 82 or 83 or 84 or 85 or 86 or 87 or 88 or 89 or 90 or 91. In some specific embodiments, the first antigen-binding moiety comprises a heavy chain variable region set forth in SEQ ID NO: 37 and a light chain variable region set forth in SEQ ID NO: 49; the second antigen-binding moiety comprises the sequence set forth in SEQ ID NO: 82 or 83 or 84 or 85 or 86 or 87 or 88 or 89 or 90 or 91. In some specific embodiments, the first antigen-binding moiety comprises a heavy chain variable region set forth in SEQ ID NO: 38 and a light chain variable region set forth in SEQ ID NO: 50; the second antigen-binding moiety comprises the sequence set forth in SEQ ID NO: 82 or 83 or 84 or 85 or 86 or 87 or 88 or 89 or 90 or 91. In some specific embodiments, the first antigen-binding moiety comprises a heavy chain variable region set forth in SEQ ID NO: 39 and a light chain variable region set forth in SEQ ID NO: 51; the second antigen-binding moiety comprises the sequence set forth in SEQ ID NO: 82 or 83 or 84 or 85 or 86 or 87 or 88 or 89 or 90 or 91. In some specific embodiments, the first antigen-binding moiety comprises a heavy chain variable region set forth in SEQ ID NO: 40 and a light chain variable region set forth in SEQ ID NO: 52; the second antigen-binding moiety comprises the sequence set forth in SEQ ID NO: 82 or 83 or 84 or 85 or 86 or 87 or 88 or 89 or 90 or 91. In some specific embodiments, the first antigen-binding moiety comprises a heavy chain variable region set forth in SEQ ID NO: 41 and a light chain variable region set forth in SEQ ID NO: 53; the second antigen-binding moiety comprises the sequence set forth in SEQ ID NO: 82 or 83 or 84 or 85 or 86 or 87 or 88 or 89 or 90 or 91. In some specific embodiments, the first antigen-binding moiety comprises a heavy chain variable region set forth in SEQ ID NO: 42 and a light chain variable region set forth in SEQ ID NO: 54; the second antigen-binding moiety comprises the sequence set forth in SEQ ID NO: 82 or 83 or 84 or 85 or 86 or 87 or 88 or 89 or 90 or 91. In some specific embodiments, the first antigen-binding moiety comprises a heavy chain variable region set forth in SEQ ID NO: 43 and a light chain variable region set forth in SEQ ID NO: 55; the second antigen-binding moiety comprises the sequence set forth in SEQ ID NO: 82 or 83 or 84 or 85 or 86 or 87 or 88 or 89 or 90 or 91. In some specific embodiments, the first antigen-binding moiety comprises a heavy chain variable region set forth in SEQ ID NO: 44 and a light chain variable region set forth in SEQ ID NO: 56; the second antigen-binding moiety comprises the sequence set forth in SEQ ID NO: 82 or 83 or 84 or 85 or 86 or 87 or 88 or 89 or 90 or 91. In some specific embodiments, the first antigen-binding moiety comprises a heavy chain variable region set forth in SEQ ID NO: 45 and a light chain variable region set forth in SEQ ID NO: 57; the second antigen-binding moiety comprises the sequence set forth in SEQ ID NO: 82 or 83 or 84 or 85 or 86 or 87 or 88 or 89 or 90 or 91. In some specific embodiments, the first antigen-binding moiety comprises a heavy chain variable region set forth in SEQ ID NO: 46 and a light chain variable region set forth in SEQ ID NO: 58; the second antigen-binding moiety comprises the sequence set forth in SEQ ID NO: 82 or 83 or 84 or 85 or 86 or 87 or 88 or 89 or 90 or 91. In some specific embodiments, the first antigen-binding moiety comprises a heavy chain variable region set forth in SEQ ID NO: 47 and a light chain variable region set forth in SEQ ID NO: 59; the second antigen-binding moiety comprises the sequence set forth in SEQ ID NO: 82 or 83 or 84 or 85 or 86 or 87 or 88 or 89 or 90 or 91.

### Monoclonal Antibody or Antigen-Binding Fragment

The present invention provides a monoclonal antibody or an antigen-binding fragment, comprising the HCDR1, HCDR2, and HCDR3 sequences of a heavy chain variable region set forth in SEQ ID NO: 36 or 37 or 38 or 39 or 40 or 41 or 42 or 43 or 44 or 45 or 46 or 47 and the LCDR1, LCDR2, and LCDR3 sequences of a light chain variable region set forth in SEQ ID NO: 48 or 49 or 50 or 51 or 52 or 53 or 54 or 55 or 56 or 57 or 58 or 59.

The present invention further provides a monoclonal antibody or an antigen-binding fragment, comprising a heavy chain variable region, wherein the heavy chain variable region comprises HCDR sequences set forth in SEQ ID NOs: 1, 2, and 3, or sequences that have one or two amino acid substitutions, deletions, or additions compared to the HCDR sequences set forth in SEQ ID NOs: 1, 2, and 3; or comprises HCDR sequences set forth in SEQ ID NOs: 4, 5, and 6, or sequences that have one or two amino acid substitutions, deletions, or additions compared to the HCDR sequences set forth in SEQ ID NOs: 4, 5, and 6; or comprises HCDR sequences set forth in SEQ ID NOs: 7, 8, and 9, or sequences that have one or two amino acid substitutions, deletions, or additions compared to the HCDR sequences set forth in SEQ ID NOs: 7, 8, and 9; or comprises HCDR sequences set forth in SEQ ID NOs: 10, 11, and 12, or sequences that have one or two amino acid substitutions, deletions, or additions compared to the HCDR sequences set forth in SEQ ID NOs: 10, 11, and 12; or comprises HCDR sequences set forth in SEQ ID NOs: 13, 14, and 15, or sequences that have one or two amino acid substitutions, deletions, or additions compared to the HCDR sequences set forth in SEQ ID NOs: 13, 14, and 15; or comprises HCDR sequences set forth in SEQ ID NOs: 16, 17, and 18, or sequences that have one or two amino acid substitutions, deletions, or additions compared to the HCDR sequences set forth in SEQ ID NOs: 16, 17, and 18. In some embodiments, the provided sequences that have one or two amino acid substitutions, deletions, or additions compared to the HCDR sequences may be obtained due to differences in calculating CDR sequences using different databases.

In some embodiments, the monoclonal antibody or the antigen-binding fragment provided further comprises a light chain variable region, wherein the light chain variable region comprises LCDR sequences set forth in SEQ ID NOs: 19, 20, and 21, or sequences that have one or two amino acid substitutions, deletions, or additions compared to the LCDR sequences set forth in SEQ ID NOs: 19, 20, and 21; or comprises LCDR sequences set forth in SEQ ID NOs: 22, 23, and 24, or sequences that have one or two amino acid substitutions, deletions, or additions compared to the LCDR sequences set forth in SEQ ID NOs: 22, 23, and 24; or comprises LCDR sequences set forth in SEQ ID NOs: 25, 20, and 26, or sequences that have one or two amino acid substitutions, deletions, or additions compared to the LCDR sequences set forth in SEQ ID NOs: 25, 20, and 26; or comprises LCDR sequences set forth in SEQ ID NOs: 27, 28, and 29, or sequences that have one or two amino acid substitutions, deletions, or additions compared to the LCDR sequences set forth in SEQ ID NOs: 27, 28, and 29; or comprises LCDR sequences set forth in SEQ ID NOs: 30, 31, and 32, or sequences that have one or two amino acid substitutions, deletions, or additions compared to the LCDR sequences set forth in SEQ ID NOs: 30, 31, and 32; or comprises LCDR sequences set forth in SEQ ID NOs: 33, 34, and 35, or sequences that have one or two amino acid substitutions, deletions, or additions compared to the LCDR sequences set forth in SEQ ID NOs: 33, 34, and 35.

The present invention further provides a monoclonal antibody or an antigen-binding fragment, comprising a heavy chain variable region and a light chain variable region, wherein the heavy chain variable region comprises complementarity-determining regions HCDR1, HCDR2, and HCDR3, and the light chain variable region comprises complementarity-determining regions LCDR1, LCDR2, and LCDR3, wherein the HCDR1 comprises the sequence set forth in SEQ ID NO: 1 or 4 or 7 or 10 or 13 or 16, the HCDR2 comprises the sequence set forth in SEQ ID NO: 2 or 5 or 8 or 11 or 14 or 17, the HCDR3 comprises the sequence set forth in SEQ ID NO: 3 or 6 or 9 or 12 or 15 or 18, the LCDR1 comprises the sequence set forth in SEQ ID NO: 19 or 22 or 25 or 27 or 30 or 33, the LCDR2 comprises the sequence set forth in SEQ ID NO: 20 or 23 or 28 or 31 or 34, and the LCDR3 comprises the sequence set forth in SEQ ID NO: 21 or 24 or 26 or 29 or 32 or 35.

In some specific embodiments, the monoclonal antibody provided has HCDR sequences set forth in SEQ ID NOs: 1, 2, and 3 and LCDR sequences set forth in SEQ ID NOs: 19, 20, and 21. In some specific embodiments, the monoclonal antibody provided has HCDR sequences set forth in SEQ ID NOs: 4, 5, and 6 and LCDR sequences set forth in SEQ ID NOs: 22, 23, and 24. In some specific embodiments, the monoclonal antibody provided has HCDR sequences set forth in SEQ ID NOs: 7, 8, and 9 and LCDR sequences set forth in SEQ ID NOs: 25, 20, and 26. In some specific embodiments, the monoclonal antibody provided has HCDR sequences set forth in SEQ ID NOs: 10, 11, and 12 and LCDR sequences set forth in SEQ ID NOs: 27, 28, and 29. In some specific embodiments, the monoclonal antibody provided has HCDR sequences set forth in SEQ ID NOs: 13, 14, and 15 and LCDR sequences set forth in SEQ ID NOs: 30, 31, and 32. In some specific embodiments, the monoclonal antibody provided has HCDR sequences set forth in SEQ ID NOs: 16, 17, and 18 and LCDR sequences set forth in SEQ ID NOs: 33, 34, and 35.

In some specific embodiments, the monoclonal antibody or the antigen-binding fragment provided comprises a heavy chain variable region that has at least 80%, 85%, or 90% sequence identity to the sequence set forth in SEQ ID NO: 36 or 37 or 38 or 39 or 40 or 41, and a light chain variable region that has at least 80%, 85%, or 90% sequence identity to the sequence set forth in SEQ ID NO: 42 or 43 or 44 or 45 or 46 or 47.

In some specific embodiments, the monoclonal antibody or the antigen-binding fragment provided is antibody m212, which comprises a heavy chain variable region set forth in SEQ ID NO: 36 and a light chain variable region set forth in SEQ ID NO: 48. In some embodiments, the monoclonal antibody or the antigen-binding fragment provided is antibody m305, which comprises a heavy chain variable region set forth in SEQ ID NO: 37 and a light chain variable region set forth in SEQ ID NO: 49. In some embodiments, the monoclonal antibody or the antigen-binding fragment provided is antibody m107, which comprises a heavy chain variable region set forth in SEQ ID NO: 38 and a light chain variable region set forth in SEQ ID NO: 50. In some embodiments, the monoclonal antibody or the antigen-binding fragment provided is antibody m202, which comprises a heavy chain variable region set forth in SEQ ID NO: 39 and a light chain variable region set forth in SEQ ID NO: 51. In some embodiments, the monoclonal antibody or the antigen-binding fragment provided is antibody m301, which comprises a heavy chain variable region set forth in SEQ ID NO: 40 and a light chain variable region set forth in SEQ ID NO: 52. In some embodiments, the monoclonal antibody or the antigen-binding fragment provided is antibody m109, which comprises a heavy chain variable region set forth in SEQ ID NO: 41 and a light chain variable region set forth in SEQ ID NO: 53. The antibody shown exhibits specific binding to GARP/TGFβ complex, e.g., strong specific binding activity to mammals including human GARP/TGFβ complex or cynomolgus monkey GARP/TGFβ complex. The antibody shown exhibits specific binding to GARP or TGFβ, e.g., strong specific binding to human, cynomolgus monkey, etc. GARP or TGFβ.

The monoclonal antibody or the antigen-binding fragment provided may also be humanized. Through humanization, the immunogenicity of the antibody or the antigen-binding fragment can be reduced, with the binding activity to the target antigen retained. In some embodiments, the monoclonal antibody or the antigen-binding fragment provided is antibody m212-hu, which comprises a heavy chain variable region set forth in SEQ ID NO: 42 and a light chain variable region set forth in SEQ ID NO: 54. In some embodiments, the monoclonal antibody or the antigen-binding fragment provided is antibody m305-hu, which comprises a heavy chain variable region set forth in SEQ ID NO: 43 and a light chain variable region set forth in SEQ ID NO: 55. In some embodiments, the monoclonal antibody or the antigen-binding fragment provided is antibody m107-hu, which comprises a heavy chain variable region set forth in SEQ ID NO: 44 and a light chain variable region set forth in SEQ ID NO: 56. In some embodiments, the monoclonal antibody or the antigen-binding fragment provided is antibody m202-hu, which comprises a heavy chain variable region set forth in SEQ ID NO: 45 and a light chain variable region set forth in SEQ ID NO: 57. In some embodiments, the monoclonal antibody or the antigen-binding fragment provided is antibody m301-hu, which comprises a heavy chain variable region set forth in SEQ ID NO: 46 and a light chain variable region set forth in SEQ ID NO: 58. In some embodiments, the monoclonal antibody or the antigen-binding fragment provided is antibody m109-hu, which comprises a heavy chain variable region set forth in SEQ ID NO: 47 and a light chain variable region set forth in SEQ ID NO: 59.

### Polynucleotide

The present invention further provides a polynucleotide encoding the bispecific antibody or the monoclonal antibody or the antigen-binding fragment. The polynucleotide mentioned is isolatable and includes, but is not limited to, DNA, RNA, cDNA, or the like. Conventional methods in the art can be used to obtain isolated polynucleotide sequences encoding bispecific antibodies or monoclonal antibodies or antigen-binding fragments.

### Construct

To prepare the antibodies mentioned in the present invention, polynucleotide sequences encoding the antibodies may be inserted into replicable expression vectors and expressed in host cells or cell-free expression systems. The present invention further provides a construct, comprising the polynucleotide described above. The construct can be obtained by a variety of methods commonly used in the art, including *in vitro* recombinant DNA technology, DNA synthesis technology, *in vivo* recombinant technology, etc.; for example, the construct can be formed by inserting the polynucleotide into the multiple cloning site of an expression vector. The construct may comprise, if desired, various operators such as a promoter, a terminator, a marker gene, etc., and these operators are operably ligated to the polynucleotide. Promoters are generally used to provide a signal for starting transcription. The lactose promoter (Lac), the Trp promoter, the Tac promoter, or the PL and PR promoters of phages may be selected, if desired. Terminators provide a signal for terminating transcription during transcription. Marker genes on constructs are often used for screening. Certainly, an enhancer may be included, if desired, to enhance protein expression. The expression vector is not particularly limited and may be some of the commercially available expression vectors, or may be an expression vector that is artificially modified if desired, e.g., a plasmid, a phage, a virus, or the like. The virus may be a plant cell virus, a mammalian cell virus, or the like. The construct can express the antibody or protein *in vitro,* and can also be transferred into a cell to express the antibody or protein.

### Host Cell

The present invention further provides a host cell, comprising the polynucleotide described above or the construct described above. Any cell suitable for antibody or protein expression of the polynucleotide or the construct may be used as a host cell. The host cell may be a prokaryotic cell, such as a bacterial cell; or a eukaryotic cell, such as a yeast cell, a mammalian cell, or the like. Host cells commonly used may be yeast cells, CHO, HEK-293 cells, COS cells, Drosophila S2, or Sf9 insect cells. The host cell comprising the polynucleotide or the construct may be obtained by methods commonly used in the art, such as microinjection, electroporation, chemical transfection, virus-mediated transformation, etc.

### Pharmaceutical Composition

The present invention further provides a pharmaceutical composition, comprising the bispecific antibody according to any of the first aspect described above, or the monoclonal antibody or the antigen-binding fragment described above, and a pharmaceutically acceptable carrier.

The pharmaceutically acceptable carrier mentioned is acceptable to the subject at the dosage or concentration employed. Pharmaceutically acceptable carriers include, but are not limited to, buffers or salts such as disodium hydrogen phosphate, sodium dihydrogen phosphate, sodium chloride, sodium acetate, citric acid, sodium citrate, and citrates, Tris; sugars such as trehalose, polysorbitol, sucrose, and mannitol; surfactants such as polysorbates; preservatives such as hexamethonium chloride, benzalkonium chloride, and benzethonium chloride; amino acids such as histidine, histidine hydrochloride, glycine, glutamine, asparagine, arginine, lysine, or the like. Sterile pharmaceutical formulations may be obtained by methods commonly used in the art, for example, by filtration through sterile filter membranes. One skilled in the art may select different pharmaceutically acceptable carriers as needed to prepare pharmaceutical compositions in different dosage forms, e.g., lyophilized dosage forms, injections, and other dosage forms. The different pharmaceutical dosage forms prepared may be formulated for administration to a subject by any suitable route of administration, including but not limited to intravenous, dermal, intramuscular, intraperitoneal, subcutaneous, nasal, oral, rectal, topical, inhalation, transdermal, etc.

### Antibody Conjugate

The present invention further provides an antibody conjugate, wherein the antibody conjugate comprises the bispecific antibody or the monoclonal antibody or the antigen-binding fragment, and a functional small molecule or a protease linked to the bispecific antibody or the monoclonal antibody or the antigen-binding fragment. The functional small molecule mentioned may be a developed or undeveloped small-molecule drug. The small-molecule drug is chemically conjugated to the bispecific antibody or the monoclonal antibody or the antigen-binding fragment to enhance the therapeutic effect of the anti-tumor drug and reduce the occurrence of adverse events. A small molecule such as a toxin, a chemotherapeutic drug, a photosensitizer, or the like may also be conjugated to the antibody mentioned by a linker. Certainly, the antibody may also be conjugated with a protac molecule or the like to obtain an antibody-Protac conjugate; for example, the antibody is linked to a protease-targeting ligand (such as an E3 ligase ligand) by a linker, so that the target protein is drawn closer to E3 ubiquitin ligase in cells and specifically degraded through the ubiquitin-proteasome pathway.

### Kit

The present invention further provides a kit, wherein the kit comprises the bispecific antibody described above or the monoclonal antibody or the antigen-binding fragment described above. The kit may further comprise, if desired, a container, a buffer, and controls such as a positive control and a negative control. One skilled in the art may make a corresponding selection if desired. Accordingly, the kit may further comprise instructions for use to facilitate the operation and use of the kit by those skilled in the art.

### Method for Producing Antibody

The present invention further provides a method for producing the bispecific antibody described above, comprising: culturing the host cell described above, and collecting the bispecific antibody from the culture.

The present invention further provides a method for producing a monoclonal antibody or an antigen-binding fragment, comprising: culturing the host cell described above, and collecting the monoclonal antibody or the antigen-binding fragment from the culture.

The bispecific antibody or the monoclonal antibody or the antigen-binding fragment collected from the culture can be purified to obtain a substantially pure product. "Substantially pure" means that the purity of the bispecific antibody or the monoclonal antibody or the antigen-binding fragment is 95% or more, 96% or more, 97% or more, 98% or more, 99% or more, or even 99.5%, 99.6%, 99.7%, 99.8% or more.

### Method for Preventing and/or Treating Disease

Furthermore, the present invention provides a method for preventing and/or treating disease, comprising: administering to a subject in need thereof an effective amount of the bispecific antibody or the monoclonal antibody or the antigen-binding fragment described above.

The "therapeutically effective amount" mentioned can result in a reduction in the severity of the symptoms of the disease, an increase in the frequency and duration of the asymptomatic phase of the disease, or prevent reductions in the suffering caused by the disease. The "prophylactically effective amount" is usually lower than the therapeutically effective amount. After being treated with the bispecific antibody or the monoclonal antibody or the antigen-binding fragment, the subject shows an inhibition rate against *in vivo* cells of 10% or more, 15% or more, 20% or more, 25% or more, 30% or more, 40% or more, 45% or more, 50% or more, 55% or more, 60% or more, 65% or more, 70% or more, 75% or more, 80% or more, 85% or more, or even 90% or more, or 95% or more, compared with a subject who has not been treated with the antibody. The subject mentioned may be an animal or a human. For example, the subject mentioned may be a mammal, including cows, sheep, mice, horses, etc.

Diseases that can be treated with the bispecific antibody or the monoclonal antibody or the antigen-binding fragment thereof provided include, but are not limited to, cancer and autoimmune diseases. In some embodiments, the method provided in the present invention can be used to treat TGF-β-related diseases, GARP-related diseases, and PD-1/PD-L1-related diseases. TGF-β-related diseases include, but are not limited to, inflammatory diseases, chronic infections, cancer, fibrosis, cardiovascular diseases, cerebrovascular diseases, neurodegenerative diseases, etc. In some embodiments, the cancer mentioned includes, but is not limited to, lung cancer, colon cancer, kidney cancer, urothelial cancer, prostate cancer, glioblastoma multiforme, ovarian cancer, pancreatic cancer, breast cancer, melanoma, liver cancer, bladder cancer, stomach cancer, esophageal cancer, hematologic cancers, etc.

The present invention further provides use of the bispecific antibody or the monoclonal antibody or the antigen-binding fragment in the preparation of a medicament or a kit. The medicament is used for treating cancer. The bispecific antibody or the monoclonal antibody or the antigen-binding fragment provided in the use can be made into a medicament for treating various diseases. It can also be used for preparing a kit and used as an immunodiagnostic reagent.

The technical solutions of the present invention are described in detail below with reference to examples. It should be noted that these examples are merely used to facilitate understanding by those skilled in the art and should not be construed as limiting the protection scope of the present invention. The examples of the present invention are described in detail below, and the described examples are illustrative, are intended to explain the present invention, and should not be construed as limiting the present invention. Methods of preparing and obtaining the sequence of the PD-L1-binding moiety derived from the second antigen-binding moiety are described in the international patent application No. PCT/CN2022/110423 (a Chinese patent application with priority application No. 202110903293.4). The international patent application No. PCT/CN2022/110423 (priority application No. 202110903293.4) is incorporated herein by reference in its entirety or in part as needed. An anti-PD-L1 nanolibrary of anti-PD-L1 nanobodies was obtained by an alpaca immunization method, and screening and identification were carried out to obtain candidate nanobodies. A fusion protein composed of the human PD-L1 protein ectodomain sequence and the human immunoglobulin Fc region sequence was mixed with Freund's adjuvant, and the mixture was emulsified and used to immunize healthy alpacas, stimulating the expression of antigen-specific nanobodies by B cells. Then blood was collected from the alpacas, and lymphocytes were separated. Total RNA was extracted using the Trizol method and reverse-transcribed into cDNA. The resulting cDNA was subjected to PCR amplification, and VHH antibody gene fragments were obtained. Competent yeast cells were transformed with the VHH gene fragments together with a yeast display vector by electroporation. The fragments and the vector were ligated by homologous recombinases of yeast to form complete plasmids. A yeast transformant library with a high insertion rate and excellent diversity was constructed and stably passaged in a nutritionally deficient culture medium. Antibody-expressing yeast cells and magnetic beads enriched with the target protein antigen were co-incubated. After several enrichment cultures, the products of magnetic bead enrichment were identified using a flow cytometer and sorted. Through several rounds of screening, 5 positive clones with high expression were obtained and designated antibody A-antibody E. The amino acid sequence of antibody A is set forth in SEQ ID NO: 82; the amino acid sequence of antibody B is set forth in SEQ ID NO: 83; the amino acid sequence of antibody C is set forth in SEQ ID NO: 84; the amino acid sequence of antibody D is set forth in SEQ ID NO: 85; the amino acid sequence of antibody E is set forth in SEQ ID NO: 86. Humanized antibodies are antibody Ahu, the amino acid sequence of which is set forth in SEQ ID NO: 87; antibody Bhu, the amino acid sequence of which is set forth in SEQ ID NO: 88; antibody Chu, the amino acid sequence of which is set forth in SEQ ID NO: 89; antibody D1hu, the amino acid sequence of which is set forth in SEQ ID NO: 90; antibody D2hu, the amino acid sequence of which is set forth in SEQ ID NO: 91.

These antibodies and humanized antibodies have high binding activity to hPD-L1 and cynomolgus monkey PD-L1, as confirmed by FACS and ELISA assays. In addition, they exhibited blocking activity against PD-1/PD-L1 and blocking activity against CD80/PD-L1.

For example, the binding activity of the antibodies to human PD-L1 was measured by FACS. The experimental process is as follows: The affinities of the different antibodies for the antigen were measured using a flow cytometer. MDA-MB-231 cells that endogenously express the PD-L1 antigen were added to a 96-well plate at 1 × 10⁵ cells/well. Samples with different concentrations were then added, and the plate was incubated at 4 °C for 30 minutes. A fluorescently labeled goat anti-human IgG secondary antibody (manufacturer: Abcam) was then added to detect antibodies bound to the cell surfaces. Antibody-antigen binding dose-response curves were generated using geometric values, and four-parameter raw data were plotted using the Graphpad Prism V6.0 software. The EC50 results for the binding of the antibodies to the antigen were determined. The experimental results show that the EC50 values for antibody A, antibody B, antibody C, antibody D, and antibody E are 0.089 nM, 0.031 nM, 0.042 nM, 0.039 nM, and 0.053 nM, respectively, which are similar to that of the positive control (atezolizumab, 0.059 nM); these antibodies exhibited high binding activity. The binding activity of the humanized antibodies was measured using a similar method. The EC50 values for antibody Ahu, antibody Bhu, antibody Chu, antibody D1hu, and antibody D2hu are 0.051 nM, 0.18 nM, 0.025 M, 0.18 nM, and 0.29 nM, respectively.

For example, the blocking activity of the antibodies against PD-1/PD-L1 was measured by FACS. The experimental process is as follows: The blocking of PD-1 and its ligand PD-L1 by the antibodies was measured using a method based on competitive flow cytometry. A 293T-PD-1 cell line that overexpresses PD-1 (manufacturer: Kyinno Biotechnology (Beijing) Co., Ltd.) was thawed, and the cells were added to a 96-well plate at 1 × 10⁵ cells/well. Antibody dilutions with different concentrations (the initial working concentration was 200 nM, and 4-fold dilution was performed) were mixed with a PD-L1-Biotin dilution (2 µg/mL, 50 µL/well), and the mixtures were incubated at room temperature for 30 minutes. Then the mixtures were added to the cells at 100 µL/well and well mixed. The plate was incubated at 4 °C for 60 minutes, then washed with FACS buffer (PBS containing 2% FBS), and centrifuged at 1200 rpm for 4 minutes, and the supernatants were discarded. PE-labeled streptavidin was added at 100 µL/well, and the plate was incubated in the dark for 30 minutes. After the incubation, the plate was washed with FACS buffer. 120 µL of FACS buffer was added to resuspend the cells, and the plate was tested on the instrument. Live cells were gated based on FSC/SSC and their mean fluorescence values were measured. Antibody-antigen blocking reaction curves were generated using geometric values, and four-parameter plots were generated using the Graphpad Prism V6.0 software. The IC50 values were determined. The experimental results show that the IC50 values for antibody A, antibody B, antibody C, antibody D, and antibody E are 1.024 nM, 2.077 nM, 1.678 nM, 2.103 nM, and 2.536nM, respectively; these antibodies can all block the binding of the antigen to its ligand. The blocking activity of the humanized antibodies was measured using a similar method. The IC50 values for antibody Ahu, antibody Bhu, antibody Chu, antibody D1hu, and antibody D2hu are 4.0 nM, 3.4 nM, 1.0 nM, 2.6 nM, and 12 nM, respectively.

The blocking activity of the antibodies against CD-80/PD-L1 was measured by FACS. The experimental process is as follows: The blocking of PD-L1 and CD-80 by the antibodies was measured using a method based on competitive flow cytometry. A 293T-PD-L1 cell line that overexpresses PD-L1 (manufacturer: Kyinno Biotechnology (Beijing) Co., Ltd.) was thawed, and the cells were added to a 96-well plate at 1 × 10⁵ cells/well. Antibody dilutions with different concentrations (the initial working concentration was 200 nM, and 4-fold dilution was performed) were added to the cells, and the plate was incubated at 4 °C for 60 minutes. After the incubation, the plate was washed with FACS buffer and centrifuged at 1200 rpm for 4 minutes, and the supernatants were discarded. Then human-CD80-mFc (2 µg/mL) was added at 100 µL/well, and the plate was incubated in the dark at 4 °C for 30 minutes. After the incubation, the plate was washed with FACS buffer. A fluorescently labeled goat anti-mouse secondary antibody (manufacturer: Abcam) diluted at 1:200 was added at 100 µL/well, and the plate was incubated in the dark at 4 °C for 30 minutes. After the incubation, the plate was washed with FACS buffer and tested on a flow cytometer. The mean fluorescence values were measured. Antibody-antigen blocking reaction curves were generated using geometric values, and four-parameter plots were generated using the Graphpad Prism V6.0 software. The IC50 values were determined. The experimental results show that the IC50 values for antibody A, antibody B, antibody C, antibody D, and antibody E are 3.954 nM, 3.355 nM, 4.146 nM, 4.757 nM, and 2.979 nM, respectively; these antibodies can all block the binding of the antigen to its ligand. The blocking activity of the humanized antibodies was measured using a similar method. The IC50 values for antibody Ahu, antibody Bhu, antibody Chu, and antibody D1hu are about 1.8 nM, 1.7 nM, 3.2 nM, and 1.5 nM, respectively.

### Example 1. Preparation of Monoclonal Antibodies

Monoclonal antibodies were obtained by hybridoma screening. Mice were immunized with human antigens (GARP, TGF-β, and GARP-TGF-β complex) to generate specific antibodies *in vivo.* Mouse myeloma cells and mouse spleen cells were fused, and the resulting cells were plated. Then HAT selection medium was added for screening. Myeloma cells did not survive, and spleen cells only survived for a very short time. Finally, only myeloma cells fused with lymphocytes survived; that is, hybridoma cells were obtained by screening.

Then hybridoma cells capable of producing specific antibodies were selected through the limiting dilution cloning method. Hybridoma cells were multi-fold diluted and inoculated onto a multi-well cell culture plate, with each well containing no more than one cell. The cells were cultured to proliferate. After the clones grew to a certain size, the supernatant in each well was assayed by ELISA for antibodies secreted by cells. Culture wells that could bind to specific antigens (GARP, TGF-β, and/or GARP-TGF-β complex) were positive wells, and dilution was continued until monoclonal cells were obtained.

More than one hundred positive clones were obtained by screening through the method described above, and these positive clones were differentially screened for clones with high activity. Finally, 15 candidate clones were selected and sequenced, and 6 of them were selected. Then the different antibodies obtained were expressed using a mammalian cell system and purified, and different antibodies with at least 90% or higher purity were obtained and were designated antibody m212, antibody m305, antibody m107, antibody m202, antibody m301, and antibody m109.

### Example 2

In Example 2, the binding activity of the antibodies to GARP and GARP-TGF-β complex was tested. Plasmids that express GARP and TGF-β were simultaneously transfected into a 293 cell line to construct a 293-GARP/TGFβ stably transfected cell system that overexpresses GARP-TGFβ complex.

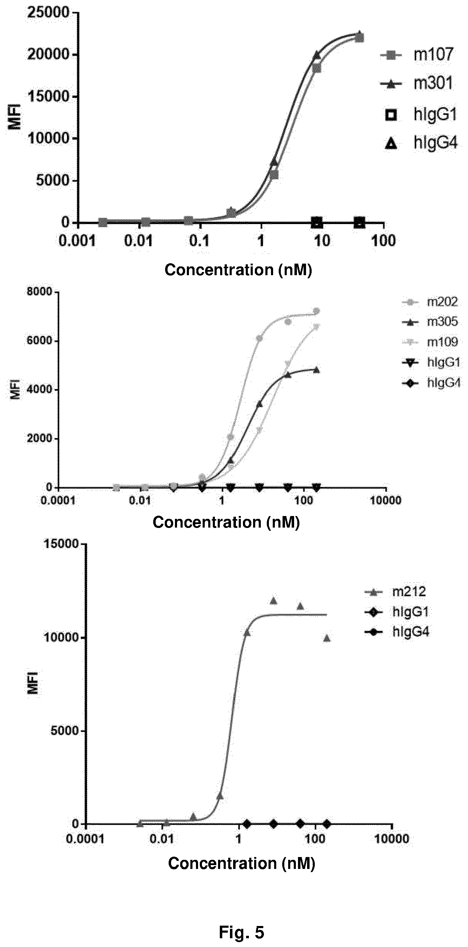

293-GARP/TGFβ cells were added to a 96-well plate at 100 µL/well, with the concentration of cells being 1-2 × 10⁶ cells/mL. The plate was washed with FACS buffer (i.e., a PBS solution containing 2% FBS). Then different antibodies to be tested (antibody m212, antibody m305, antibody m107, antibody m202, antibody m301, and antibody m109; the initial concentration was 200 nM, and 4-fold gradient dilution was performed) were added at 100 µL/well, and the plate was incubated at 4 °C for 30 min. The plate was washed with buffer. A fluorescently labeled goat anti-human secondary antibody (manufacturer: Abcam) was added, and the plate was incubated at 4 °C for 30 min. The plate was washed with buffer, and buffer was added to resuspend the cells. Fluorescent signals were detected by a flow cytometer. EC50 values for the test samples were calculated using the data processing software Graphpad Prism 6.0. The results are shown in Table 1 below and FIG. 5.

**Table 1**

| Antibody No. | 293-GARP/TGF β cell EC50 (nM) |
|---|---|
| m212 | <2 |
| m305 | 4.1 |
| m107 | 3.2 |
| m202 | 2.7 |
| m301 | 2.5 |
| m109 | >5 |
| hIgG1 | No binding |
| hIgG4 | No binding |

As can be seen from the results in Table 1 and FIG. 5, antibody m212, antibody m305, antibody m107, antibody m202, antibody m301, and antibody m109 can all bind to GARP-TGF-β complex.

GARP-expressing plasmids were transfected into the CHO-K1 cell line to construct a CHO-K1-GARP cell line that overexpresses GARP. CHO-K1-GARP cells were added to a 96-well plate at 100 µL/well, with the concentration of cells being 1-2 × 10⁶ cells/mL. The plate was washed with FACS buffer (i.e., a PBS solution containing 2% FBS). Then different antibodies to be tested (taking antibody m212, antibody m305, and antibody m107 as examples; the initial concentration was 200 nM, and 4-fold gradient dilution was performed) were added at 100 µL/well, and the plate was incubated at 4 °C for 30 min. The plate was washed with buffer. A fluorescently labeled goat anti-human secondary antibody (manufacturer: Abcam) was added, and the plate was incubated at 4 °C for 30 min. The plate was washed with buffer, and buffer was added to resuspend the cells. Fluorescent signals were detected by a flow cytometer. EC50 values for the test samples were calculated using the data processing software Graphpad Prism 6.0. The results are shown in Table 2 below.

**Table 2**

| Antibody No. | CHO-K1-GARP cell EC50 (nM) |
|---|---|
| m212 | 0.46 |
| m305 | <1 |
| m107 | >2 |
| hIgG1 | No binding |
| hIgG4 | No binding |

As can be seen from Table 2, antibody m212, antibody m305, and antibody m107 can all specifically bind to GARP protein.

### Example 3

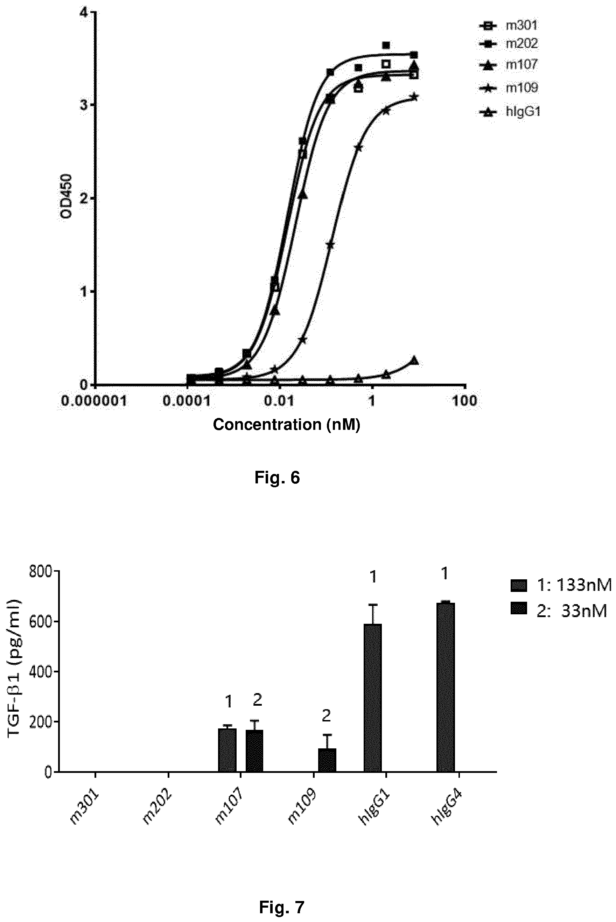

In Example 3, the binding activity of the antibodies to TGF-β was identified. A cell culture plate was coated with human transforming growth factor β1 (hTGF-β1, manufacturer: ACRO) at 100 ng/well and incubated overnight at 4 °C. Then the plate was washed with PBST. BSA was added at 200 µL/well, and the plate was blocked at 37 °C for 1 h. The plate was washed with PBST, and different concentrations of antibodies to be tested (taking antibody m107, antibody m202, antibody m301, and antibody m109 as examples; the initial concentration for each of the antibodies was 31.25 nM, and 4-fold gradient dilution was performed) were added at 100 µL/well. The plate was incubated at 37 °C for 2 h. The plate was washed with PBST. A goat anti-human secondary antibody (diluted with 1% BSA at 1:10,000, manufacturer: Jackson) was added at 100 µL/well, and the plate was incubated at 37 °C for 1 h. The plate was washed with PBST, and 100 µL of TMB substrate solution was added. After 10-15 min of color development, 50 µL of 2 M sulfuric acid was added to terminate the reaction. OD450 values were measured using a microplate reader, and EC50 values for the test antibodies were calculated using the data processing software Graphpad Prism 6.0. The results are shown in Table 3 below and FIG. 6.

**Table 3**

| Antibody No. | EC50 (nM) |
|---|---|
| m107 | 0.021 |
| m202 | <1 |
| m301 | <1 |
| m109 | <1 |
| hIgG1 | No binding |

The experimental results show that antibody m107, antibody m202, antibody m301, and antibody m109 can all specifically bind to TGF-β.

### Example 4. TGF-β Secretion and Neutralization Assay

Anti-CD3 antibodies can bind to the TCR complex, activating T lymphocytes and mediating T lymphocytes' secretion of TGF-β; anti-CD28 antibodies can work with anti-CD3 antibodies to activate and expand T lymphocytes *in vitro.* Anti-CD3 and CD28 antibodies can be used to synergistically stimulate and activate immune cells.

1 µg/mL solutions of anti-CD3 antibody and anti-CD28 antibody (manufacturer: Biolegend) were prepared and added to a 96-well plate at 100 µL/well, and the plate was coated overnight at 4 °C. Then Tregs (the concentration of cells was 5 × 10⁵ cells/mL) were added at 100 µL/well. Different concentrations (266 nM and 66 nM) of the antibodies to be tested were added at 100 µL/well, and the plate was incubated in an incubator for 96 h. Finally, the TGF-β content of the supernatants was determined using a TGF-β ELISA assay kit (manufacturer: BD). Analysis was performed using the data processing software Graphpad Prism 6.0, and experimental results were obtained. The experimental results are shown in FIG. 7.

As can be seen from FIG. 7, taking antibody m202, antibody m301, antibody m109, and antibody m107 as examples, these antibodies can all inhibit or neutralize TGF-β produced by Tregs.

### Example 5

Humanized antibodies were obtained by the CDR-grafting method, which fulfills the purpose of humanization by dividing the amino acid sequences of an antibody and a template into FRs and CDRs in the same way and then grafting the CDRs onto the FRs of the template so that the CDRs that determine the specificity of the antibody are combined with the framework regions of a human antibody. Meanwhile, in order to avoid reductions in the affinity of the antibodies, key amino acid residue positions were obtained by calculations through computer simulation and other technologies, and a back mutation method was used to ensure the affinities of the humanized antibodies. As designed, the antibody obtained by humanization of m212 was numbered m212-hu; the antibody obtained by humanization of m305 was numbered m305-hu; the antibody obtained by humanization of m107 was numbered m107-hu; the antibody obtained by humanization of m202 was numbered m202-hu; the antibody obtained by humanization of m301 was numbered m301-hu; and the antibody obtained by humanization of 109 was numbered m109-hu. Then the activity of each of these humanized antibodies was measured.

The 293-GARP/TGFβ cell strain and the CHO-K1-GARP cell line were separately added to a 96-well cell plate at 100 µL/well, with the concentration of cells being 1-2 × 10⁶ cells/mL. The plate was washed with FACS buffer (2% FBS). Then different concentrations of the antibodies to be tested (the initial concentration was 200 nM, and 4-5 fold gradient dilution was performed) were added at 100 µL/well, and the plate was incubated at 4 °C for 30 min. The plate was washed with FACS buffer. A fluorescently labeled goat anti-human secondary antibody was added, and the plate was incubated at 4 °C for 30 min. The plate was washed with FACS buffer, and 150-200 µL of FACS buffer was added to resuspend the cells. Fluorescent signals were detected by a flow cytometer. EC50 values for the test samples were calculated using the data processing software Graphpad Prism 6.0. The results are shown in Table 4 and Table 5 below.

**Table 4**

| No. | GARP-TGF-β complex EC50 (nM) |
|---|---|
| m212-hu | <2 |
| m305-hu | 2.1 |
| m107-hu | 7.5 |
| m202-hu | 2.5 |
| m301-hu | 4.0 |
| m109-hu | >5 |
| hIgG1 | No binding |
| hIgG4 | No binding |

Taking the above antibodies as examples, the humanized antibodies exhibited strong specific binding activity to GARP-TGF-β complex.

**Table 5**

| No. | GARP EC50 (nM) |
|---|---|
| m212-hu | 1.4 |
| m305-hu | 0.45 |
| hIgG1 | No binding |
| hIgG4 | No binding |

Taking the above antibodies as examples, the experimental results show that these antibodies exhibited strong binding activity to GARP protein.

A microplate was coated with hTGF-β1 (manufacturer: ACRO) at 100 ng/well and incubated overnight at 4 °C. Then the plate was washed with PBST. BSA was added at 200 µL/well, and the plate was blocked at 37 °C for 1 h. The plate was washed with PBST. Different concentrations of the antibodies to be tested (the initial concentration was 31.25 nM, and 4-fold gradient dilution was performed with 1% BSA) were added at 100 µL/well, and the plate was incubated at 37 °C for 2 h. The plate was washed with PBST. Then a goat anti-human secondary antibody (diluted with 1% BSA at 1:10,000) was added at 100 µL/well, and the plate was incubated at 37 °C for 1 h. The plate was washed with PBST, and 100 µL of TMB substrate solution was added. After 10-15 min of color development, 50 µL of 2 M sulfuric acid was added to terminate the reaction. OD450 values were measured using a microplate reader, and EC50 values for the test antibodies were calculated using the data processing software Graphpad Prism 6.0. The results are shown in Table 6 below.

**Table 6**

| No. | EC50 (nM) |
|---|---|
| m107-hu | 7.5 |
| m202-hu | <2 |
| m301-hu | <2 |
| m109-hu | 0.73 |
| hIgG1 | No binding |

The experimental results show that, taking the above antibodies as examples, the humanized antibodies also exhibited specific binding activity to TGF-β. Furthermore, the neutralizing activity of the humanized antibodies against TGF-β was measured using the method mentioned in Example 4. The results show that the antibodies can all specifically bind to TGF-β and neutralize TGF-β.

### Example 6

This example mainly describes the genetic engineering of anti-human GARP or anti-GARP-TGF-β complex antibodies and anti-PD-L1 antibodies to construct and express anti-GARP and PD-L1 or anti-TGF-β, GARP-TGF-β complex, and PD-L1 bispecific antibodies. The bispecific antibodies were named starting with b so as to be distinguished from mAbs with names that start with m. The bispecific antibodies were formed by linking using the linker (G₄S)₄. In the process of linking, if an anti-PD-L1 nanobody is linked to the third heavy chain constant domain of an IgG-type structure, as shown in FIG. 1, then the names of the two antigen-binding moieties are joined by H (for distinction), and the two antigen-binding moieties retain their original reference numbers. For example, "b212-hu-H-D2hu" represents a bispecific antibody formed by linking the sequence of antibody m212-hu and the sequence of the anti-PD-L1 nanobody D2hu, and the nanobody is linked to the third heavy chain constant domain of the IgG-type structure. When nanobodies are linked to the third heavy chain constant domain of an IgG-type structure, if the nanobody moieties are indicated by only one reference number, it means that the nanobodies linked to the carboxy-termini are identical and the nanobodies linked to the two symmetrical carboxy-termini of the third heavy chain constant domain are identical; if the nanobodies linked are different, the reference numbers of all the nanobodies linked should be shown. For example, "b301-H-Bhu-D2hu" represents a bispecific antibody formed by linking the sequence of antibody m301-hu and the sequences of the anti-PD-L1 nanobodies Bhu and D2hu, and Bhu and D2hu are linked to the carboxy-termini of the third heavy chain constant domain of the IgG-type structure, respectively.

Referring to FIG. 3, in the process of linking, if anti-PD-L1 nanobodies are linked to the light chain constant domains of an IgG-type structure, respectively (what is different from FIG. 3 is that each of the C-termini of the light chain constant domains is linked to one anti-PD-L1 nanobody), then the names of the two antigen-binding moieties are joined by L (for distinction). Referring to FIG. 2, in the process of linking, the carboxy-termini of the heavy chain constant domains of an IgG-type structure may be symmetrically linked to two anti-PD-L1 nanobodies. Taking any of the carboxy-termini of the heavy chain constant domains as an example, if the two nanobodies to which it is linked are different, then the names of the two nanobodies are joined by "-", and the two nanobodies to which it is linked are indicated by the reference numbers of the nanobodies; the two nanobodies to which it is linked may be linked by (G₄S)₃.

Subsequently, bispecific antibody proteins were produced by transient transfection of CHO-S cells. The supernatants were collected, and the antibodies were purified through a protein A affinity column. The purity of the purified antibodies was measured by HPLC and SDS-PAGE; the purity of the bispecific antibodies was 90% or above.

### Example 7. Binding Activity of Bispecific Antibodies

A 293-GARP/TGFβ cell strain, a CHO-K1-GARP cell strain, and an MC38-hPD-L1 (manufacturer: Kyinno Biotechnology (Beijing) Co., Ltd.) cell line were cultured. The concentration of cells was adjusted to 1-2 × 10⁶ cells/mL, and the cells were added to a 96-well cell plate at 100 µL/well. Then different concentrations of the antibodies to be tested (the initial concentration was 200 nM, and 4-5 fold gradient dilution was performed) were added at 100 µL/well, and the plate was incubated at 4 °C for 30 min. The plate was washed with FACS buffer. A fluorescently labeled goat anti-human secondary antibody was added, and the plate was incubated at 4 °C for 30 min. The plate was washed with FACS buffer, and 150-200 µL of FACS buffer was added to resuspend the cells. Fluorescent signals were detected by a flow cytometer. EC50 values for the test antibodies were calculated using the data processing software Graphpad Prism 6.0. The results are shown in Tables 7-9 below.

**Table 7. The results for the binding of different antibodies to the 293-GARP/TGFβ cell strain**

| Antibody No. | EC50 (nM) |
|---|---|
| b301-H- Bhu-D2hu | 1.3 |
| b301-L-Bhu | 4.4 |
| b202-H-Bhu-D2hu | 1.5 |
| b202-L-Bhu | 1.6 |
| b305-H-Bhu-D2hu | 4.4 |
| b212-L-D2hu | 7.7 |
| b212-hu-H-D2hu | 7.7 |
| b212-L-Bhu | 1.9 |
| b202-H-D2hu | 5.6 |
| b202-hu-L-D2hu | 5.4 |
| b301-H-D2hu | 7.3 |
| b301-L-D2hu | 11 |
| hIgG1 | No binding |

**Table 8. The results for the binding of different antibodies to the CHO-K1-GARP cell strain**

| Antibody No. | EC50 (nM) |
|---|---|
| b212-L-D2hu | 1.9 |
| b212-hu-H-D2hu | 2.2 |
| b212-L-Bhu | 2.0 |

**Table 9. The results for the binding of different antibodies to the MC38-hPD-L1 cell strain**

| Antibody No. | EC50 (nM) |
|---|---|
| m212-hu | No binding |
| b212-L-D2hu | 0.39 |
| b212-hu-H-D2hu | 0.46 |
| b212-L-Bhu | 0.59 |
| m202-hu | No binding |
| b202-L-D2hu | 0.39 |
| b202-hu-H-D2hu | 0.34 |
| m301-hu | No binding |
| b301-H-D2hu | 0.34 |
| b301-L-D2hu | 0.52 |
| hIgG1 | No binding |

### Example 8. TGFβ Reporter Gene Blocking Activity

293F-GAPR/TGFβ/αvβ6 cells were cultured, and the cells (cell density of 0.6 × 10⁶ cells/mL) were added to a cell culture plate at 50 µL/well, and 293-SBE-Luciferase cells (cell density of 1.8 × 10⁶ cells/mL) were then added to the cell culture plate at 50 µL/well. Then different concentrations of the antibodies to be tested (the initial concentration was 400 nM or 133.33 nM, and 3-fold gradient dilution was performed) were added to the cell culture plate at 100 µL/well. After a thorough mixing, the plate was incubated in an incubator for 18-24 h. The luciferase substrate ONE-Glo^{™} Luciferase Assay system was added at 100 µL/well, and the plate was incubated in the dark for 5 min. Then luminescent signal values were measured using a microplate reader. IC50 values for the test samples were calculated using the data processing software Graphpad Prism 6.0. The results are shown in Table 10 below.

**Table 10**

| Antibody No. | IC50(nM) |
|---|---|
| b212-L-D2hu | 0.38 |
| b212-hu-H-D2hu | 0.38 |
| b202-hu-H-D2hu | 4.9 |
| b202-L-D2hu | 1.7 |
| b301-H-D2hu | 4.2 |
| b301-L-D2hu | 8.4 |
| hIgG1 | No blocking |

Taking the above BsAbs as examples, the results show that the above BsAbs exhibited blocking activity against TGF-β.

### Example 9. PD-L1 Antibody Reporter Gene Blocking System

In Example 9, the blocking of the PD-L1/PD-1 Pathway by the antibodies was studied through a reporter gene experiment. The experiment was completed with two cell lines: a Jurkat-PD1-CD3zeta-NFAT-Luc2 cell strain (manufacturer: Kyinno Biotechnology (Beijing) Co., Ltd.), in which a fusion protein consisting of PD-1 ECD and CD3zeta is stably expressed in Jurkat cells and a luciferase reporter gene driven by NF-AT is inserted; and 293T-hPD-L1 cells (manufacturer: Kyinno Biotechnology (Beijing) Co., Ltd.), i.e., 293T cells that express human PD-L1. When the two types of cells are co-cultured, the interaction of PD-1/PD-L1 serves as a first signal, and the intracellular CD3zeta chain serves as a second signal; activation signals are transmitted inward, enabling the expression of the NFAT-driven luciferase reporter gene, which results in the emission of fluorescent light. When a PD-L1 antibody is added, the PD-1/PD-L1 binding is blocked, the transmission of activation signals is suppressed, and the luciferase reporter gene cannot be expressed.

293T-hPD-L1 cells were added to a 96-well plate at 2 × 10⁴ cells/well, and Jurkat-PD1-CD3zeta-NFAT-Luc2 effector cells were then added to the 96-well plate at 2 × 10⁴ cells/well. Then different concentrations of the antibodies to be tested (the initial concentration was 60 µg/mL, and 4-fold gradient dilution was performed) were added, and the plate was incubated at 37 °C for 18-24 h. The luciferase substrate ONE-Glo^{™} Luciferase Assay system assay reagent was added at 100 µL/well, and the plate was incubated in the dark for 5 minutes. Fluorescent signals in the 96-well plate were measured using a microplate reader. Four-parameter curves were generated with the y-axis representing the relative fluorescence value and the x-axis representing the antibody sample concentration. The curves were analyzed using the GraphPad Prism 6.0 software, and the IC50 values for the BsAbs were obtained. The results are shown in Table 11 below. Taking the shown antibodies as examples, the mAbs did not exhibit blocking activity against PD-L1, and the BsAbs exhibited blocking activity against PD-L1. Referring to the method of Example 5, these antibodies also exhibited neutralizing activity against TGF-β.

**Table 11**

| Antibody No. | IC50(nM) |
|---|---|
| m212-hu | No blocking |
| b212-L-D2hu | 8.2 |
| b212-hu-H-D2hu | 8.7 |
| m202-hu | No blocking |
| b202-L-D2hu | 13 |
| b202-hu-H-D2hu | 7.8 |
| m301-hu | No blocking |
| b301-H-D2hu | 11 |
| b301-L-D2hu | 10 |
| hIgG1 | No blocking |

### Example 10. Evaluation of In-Vivo Activity

MC38-hPD-L1KL#3 cells were constructed, with mouse PD-L1 knocked out and the human PD-L1 gene inserted. For experimentation, MC-38-hPD-L1 KL#3 cells were taken and subcutaneously inoculated into the right scapula sites of C57BL/6-hGARP transgenic female mice (manufacturer: GemPharmatech). The inoculation volume was 0.1 mL/mouse, and the cell density was 1 × 10⁶ cells/mouse. When the tumor volume reached 75-100 mm³, the mice were grouped, as shown in Table 12. The experimental mice were divided into 5 groups of 6 and were intraperitoneally administered drugs 4 times, twice a week. After administration, the body weight of the mice and the tumor volume were measured every 3-4 days.

**Table 12**

| Group | Drug group | Dose (mg/kg) | Administration volume parameter 100 µL/25 g (µL/g) | Route of administration | Frequency of administration | Period of administration |
|---|---|---|---|---|---|---|
| 1 | Vehicle (vehicle group, PBS) | NA | 4 | i.p. | BIW | 2 weeks |
| 2 | b202-hu-H-D2hu | 4.4 | 4 | i.p. | BIW | 2 weeks |
| 3 | m202-hu + D2hu (combination administration) | 3.7+2 | 4 | i.p. | BIW | 2 weeks |
| 4 | b301-hu-H-D2hu | 4.4 | 4 | i.p. | BIW | 2 weeks |
| 5 | m301-hu | 3.7 | 4 | i.p. | BIW | 2 weeks |
| 6 | m302-hu | 3.7 | 4 | i.p. | BIW | 2 weeks |

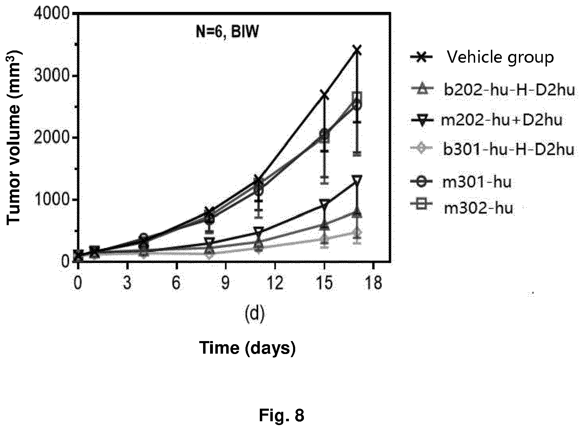

The results of the animal experiment are shown in FIG. 8. The results show that the shown antibodies all exhibited anti-tumor effects and that the tumor size and volume were inhibited; for example, the tumor growth inhibition (TGI) results are between 45% and 60%. Moreover, the BsAbs exhibited significantly better anti-tumor effects than the mAbs and can achieve a tumor growth inhibition of 75% or above.

In the description of the present invention, unless otherwise clearly and specifically defined, "a plurality of" means at least two, e.g., two, three, etc.

In the specification, the reference term "an embodiment", "some embodiments", "an example", "a specific example", or "some examples" means that a specific feature, structure, material, or characteristic described in connection with the embodiment or example is included in at least one embodiment or example of the present invention. In the specification, exemplary descriptions of the foregoing terms are not necessarily intended for the same embodiment or example. Moreover, the specific features, structures, materials, or characteristics described may be combined in any one or more embodiments or examples in a suitable manner. In addition, one skilled in the art may integrate or combine different embodiments or examples described in the specification and features of the different embodiments or examples so long as they are not contradictory to each other.

Although the embodiments of the present disclosure have been shown and described above, it can be understood that the foregoing embodiments are exemplary and should not be understood as limitations on the present invention. A person of ordinary skill in the art can make changes, modifications, replacements, and variations to the foregoing embodiments within the scope of the present invention.

## Claims

1. A bispecific antibody, wherein the bispecific antibody comprises a first antigen-binding moiety and a second antigen-binding moiety;
the first antigen-binding moiety comprises a heavy chain variable region, and the heavy chain variable region comprises:
HCDR sequences set forth in SEQ ID NOs: 1, 2, and 3, or sequences that have one or two amino acid substitutions, deletions, or additions compared to the HCDR sequences set forth in SEQ ID NOs: 1, 2, and 3;
or HCDR sequences set forth in SEQ ID NOs: 4, 5, and 6, or sequences that have one or two amino acid substitutions, deletions, or additions compared to the HCDR sequences set forth in SEQ ID NOs: 4, 5, and 6;
or HCDR sequences set forth in SEQ ID NOs: 7, 8, and 9, or sequences that have one or two amino acid substitutions, deletions, or additions compared to the HCDR sequences set forth in SEQ ID NOs: 7, 8, and 9;
or HCDR sequences set forth in SEQ ID NOs: 10, 11, and 12, or sequences that have one or two amino acid substitutions, deletions, or additions compared to the HCDR sequences set forth in SEQ ID NOs: 10, 11, and 12;
or HCDR sequences set forth in SEQ ID NOs: 13, 14, and 15, or sequences that have one or two amino acid substitutions, deletions, or additions compared to the HCDR sequences set forth in SEQ ID NOs: 13, 14, and 15;
or HCDR sequences set forth in SEQ ID NOs: 16, 17, and 18, or sequences that have one or two amino acid substitutions, deletions, or additions compared to the HCDR sequences set forth in SEQ ID NOs: 16, 17, and 18;
the first antigen-binding moiety further comprises a light chain variable region, and the light chain variable region comprises:
LCDR sequences set forth in SEQ ID NOs: 19, 20, and 21, or sequences that have one or two amino acid substitutions, deletions, or additions compared to the LCDR sequences set forth in SEQ ID NOs: 19, 20, and 21;
or LCDR sequences set forth in SEQ ID NOs: 22, 23, and 24, or sequences that have one or two amino acid substitutions, deletions, or additions compared to the LCDR sequences set forth in SEQ ID NOs: 22, 23, and 24;
or LCDR sequences set forth in SEQ ID NOs: 25, 20, and 26, or sequences that have one or two amino acid substitutions, deletions, or additions compared to the LCDR sequences set forth in SEQ ID NOs: 25, 20, and 26;
or LCDR sequences set forth in SEQ ID NOs: 27, 28, and 29, or sequences that have one or two amino acid substitutions, deletions, or additions compared to the LCDR sequences set forth in SEQ ID NOs: 27, 28, and 29;
or LCDR sequences set forth in SEQ ID NOs: 30, 31, and 32, or sequences that have one or two amino acid substitutions, deletions, or additions compared to the LCDR sequences set forth in SEQ ID NOs: 30, 31, and 32;
or LCDR sequences set forth in SEQ ID NOs: 33, 34, and 35, or sequences that have one or two amino acid substitutions, deletions, or additions compared to the LCDR sequences set forth in SEQ ID NOs: 33, 34, and 35;
the first antigen-binding moiety is capable of binding to TGF-β, GARP, or GARP-TGF-β complex, and the second antigen-binding moiety is capable of binding to PD-L1;
the HCDR sequences and the LCDR sequences are obtained based on the IMGT definition scheme.

2. The bispecific antibody according to claim 1, wherein the first antigen-binding moiety has:
(1) HCDR sequences set forth in SEQ ID NOs: 1, 2, and 3 or sequences that have one or two amino acid substitutions, deletions, or additions compared to the HCDR sequences set forth in SEQ ID NOs: 1, 2, and 3; and LCDR sequences set forth in SEQ ID NOs: 19, 20, and 21 or sequences that have one or two amino acid substitutions, deletions, or additions compared to the LCDR sequences set forth in SEQ ID NOs: 19, 20, and 21; or
(2) HCDR sequences set forth in SEQ ID NOs: 4, 5, and 6 or sequences that have one or two amino acid substitutions, deletions, or additions compared to the HCDR sequences set forth in SEQ ID NOs: 4, 5, and 6; and LCDR sequences set forth in SEQ ID NOs: 22, 23, and 24 or sequences that have one or two amino acid substitutions, deletions, or additions compared to the LCDR sequences set forth in SEQ ID NOs: 22, 23, and 24; or
(3) HCDR sequences set forth in SEQ ID NOs: 7, 8, and 9 or sequences that have one or two amino acid substitutions, deletions, or additions compared to the HCDR sequences set forth in SEQ ID NOs: 7, 8, and 9; and LCDR sequences set forth in SEQ ID NOs: 25, 20, and 26 or sequences that have one or two amino acid substitutions, deletions, or additions compared to the LCDR sequences set forth in SEQ ID NOs: 25, 20, and 26; or
(4) HCDR sequences set forth in SEQ ID NOs: 10, 11, and 12 or sequences that have one or two amino acid substitutions, deletions, or additions compared to the HCDR sequences set forth in SEQ ID NOs: 10, 11, and 12; and LCDR sequences set forth in SEQ ID NOs: 27, 28, and 29 or sequences that have one or two amino acid substitutions, deletions, or additions compared to the LCDR sequences set forth in SEQ ID NOs: 27, 28, and 29; or
(5) HCDR sequences set forth in SEQ ID NOs: 13, 14, and 15 or sequences that have one or two amino acid substitutions, deletions, or additions compared to the HCDR sequences set forth in SEQ ID NOs: 13, 14, and 15; and LCDR sequences set forth in SEQ ID NOs: 30, 31, and 32 or sequences that have one or two amino acid substitutions, deletions, or additions compared to the LCDR sequences set forth in SEQ ID NOs: 30, 31, and 32; or
(6) HCDR sequences set forth in SEQ ID NOs: 16, 17, and 18 or sequences that have one or two amino acid substitutions, deletions, or additions compared to the HCDR sequences set forth in SEQ ID NOs: 16, 17, and 18; and LCDR sequences set forth in SEQ ID NOs: 33, 34, and 35 or sequences that have one or two amino acid substitutions, deletions, or additions compared to the LCDR sequences set forth in SEQ ID NOs: 33, 34, and 35.

3. A bispecific antibody, wherein the bispecific antibody comprises a first antigen-binding moiety and a second antigen-binding moiety;
the first antigen-binding moiety comprises a heavy chain variable region and a light chain variable region; the heavy chain variable region comprises complementarity-determining regions HCDR1, HCDR2, and HCDR3, and the light chain variable region comprises complementarity-determining regions LCDR1, LCDR2, and LCDR3, wherein:
the HCDR1 comprises the sequence set forth in SEQ ID NO: 1 or 4 or 7 or 10 or 13 or 16;
the HCDR2 comprises the sequence set forth in SEQ ID NO: 2 or 5 or 8 or 11 or 14 or 17;
the HCDR3 comprises the sequence set forth in SEQ ID NO: 3 or 6 or 9 or 12 or 15 or 18;
the LCDR1 comprises the sequence set forth in SEQ ID NO: 19 or 22 or 25 or 27 or 30 or 33;
the LCDR2 comprises the sequence set forth in SEQ ID NO: 20 or 23 or 28 or 31 or 34;
the LCDR3 comprises the sequence set forth in SEQ ID NO: 21 or 24 or 26 or 29 or 32 or 35;
the first antigen-binding moiety is capable of binding to TGF-β, GARP, or GARP-TGF-β complex, and the second antigen-binding moiety is capable of binding to PD-L1;
the HCDR1, HCDR2, and HCDR3 sequences and the LCDR1, LCDR2, and LCDR3 sequences are obtained based on the IMGT definition scheme.

4. The bispecific antibody according to any of claims 1-3, wherein the first antigen-binding moiety comprises:
a heavy chain variable region that has at least 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence set forth in SEQ ID NO: 36 or 37 or 38 or 39 or 40 or 41 or 42 or 43 or 44 or 45 or 46 or 47, and
a light chain variable region that has at least 80%, 81%, 82%, 83%, 84%, 85%, 86%, 87%, 88%, 89%, 90%, 91%, 1%, 92%, 93%, 94%, 95%, 96%, 97%, 98%, or 99% sequence identity to the sequence set forth in SEQ ID NO: 48 or 49 or 50 or 51 or 52 or 53 or 54 or 55 or 56 or 57 or 58 or 59.

5. The bispecific antibody according to claim 4, wherein the first antigen-binding moiety comprises:
(1) a heavy chain variable region set forth in SEQ ID NO: 36 or 37 or 38 or 39 or 40 or 41, and a light chain variable region set forth in SEQ ID NO: 48 or 49 or 50 or 51 or 52 or 53; or
(2) a heavy chain variable region set forth in SEQ ID NO: 42 or 43 or 44 or 45 or 46 or 47, and a light chain variable region set forth in SEQ ID NO: 54 or 55 or 56 or 57 or 58 or 59.

6. The bispecific antibody according to any of claims 1-5, wherein the second antigen-binding moiety is a single-domain antibody;
the bispecific antibody is a structure in which an IgG-type antibody is linked to the second antigen-binding moiety by a linker, wherein the IgG-type antibody comprises the first antigen-binding moiety.

7. The bispecific antibody according to any of claims 1-5, wherein the second antigen-binding moiety comprises:
(a) a CDR1, the CDR1 comprising a sequence selected from the group consisting of SEQ ID NOs: 60, 63, and 66, or sequences that have one or two amino acid substitutions, deletions, or additions compared to SEQ ID NOs: 60, 63, and 66;
(b) a CDR2, the CDR2 comprising a sequence selected from the group consisting of SEQ ID NOs: 61, 64, and 67, or sequences that have one or two amino acid substitutions, deletions, or additions compared to SEQ ID NOs: 61, 64, and 67;
and
(c) a CDR3, the CDR3 comprising a sequence selected from the group consisting of SEQ ID NOs: 62, 65, 68, and 69, or sequences that have one, two, or three amino acid substitutions, deletions, or additions compared to SEQ ID NOs: 62, 65, 68, and 69;
the CDR1, CDR2, and CDR3 are obtained based on the IMGT definition scheme.

8. The bispecific antibody according to claim 7, wherein the second antigen-binding moiety is selected from:
(1) a sequence that has a CDR1 sequence set forth in SEQ ID NO: 60, a CDR2 sequence set forth in SEQ ID NO: 61, and a CDR3 sequence set forth in SEQ ID NO: 62; or
(2) a sequence that has a CDR1 sequence set forth in SEQ ID NO: 63, a CDR2 sequence set forth in SEQ ID NO: 64, and a CDR3 sequence set forth in SEQ ID NO: 65; or
(3) a sequence that has a CDR1 sequence set forth in SEQ ID NO: 66, a CDR2 sequence set forth in SEQ ID NO: 67, and a CDR3 sequence set forth in SEQ ID NO: 68; or
(4) a sequence that has a CDR1 sequence set forth in SEQ ID NO: 60, a CDR2 sequence set forth in SEQ ID NO: 61, and a CDR3 sequence set forth in SEQ ID NO: 69.

9. The bispecific antibody according to claim 7 or 8, wherein the second antigen-binding moiety further comprises framework regions, including an FR1, an FR2, an FR3, and an FR4; the FR1, FR2, FR3, and FR4 comprise the following amino acid sequences:
(1) the FR1 comprises a sequence selected from the sequence set forth in SEQ ID NO: 70 or 74 or 77 or 80, or a sequence that has one, two, or three conservative amino acid substitutions compared to the sequence set forth in SEQ ID NO: 70 or 74 or 77 or 80;
(2) the FR2 comprises a sequence selected from the sequence set forth in SEQ ID NO: 71 or 75 or 78, or a sequence that has one, two, or three conservative amino acid substitutions compared to the sequence set forth in SEQ ID NO: 71 or 75 or 78;
(3) the FR3 comprises a sequence selected from the sequence set forth in SEQ ID NO: 72 or 76 or 79 or 81, or a sequence that has one, two, three, or four conservative amino acid substitutions compared to the sequence set forth in SEQ ID NO: 72 or 76 or 79 or 81;
(4) the FR4 is selected from the sequence set forth in SEQ ID NO: 73, or a sequence that has one or two conservative amino acid substitutions compared to the sequence set forth in SEQ ID NO: 73.

10. The bispecific antibody according to claim 7 or 8, wherein the second antigen moiety further comprises framework regions (FRs), and the framework regions (FRs) are selected from:
(1) an FR1 sequence set forth in SEQ ID NO: 70, an FR2 sequence set forth in SEQ ID NO: 71, an FR3 sequence set forth in SEQ ID NO: 72, and an FR4 sequence set forth in SEQ ID NO: 73; or
(2) an FR1 sequence set forth in SEQ ID NO: 74, an FR2 sequence set forth in SEQ ID NO: 75, an FR3 sequence set forth in SEQ ID NO: 76, and an FR4 sequence set forth in SEQ ID NO: 73; or
(3) an FR1 sequence set forth in SEQ ID NO: 77, an FR2 sequence set forth in SEQ ID NO: 78, an FR3 sequence set forth in SEQ ID NO: 79, and an FR4 sequence set forth in SEQ ID NO: 73; or
(4) an FR1 sequence set forth in SEQ ID NO: 80, an FR2 sequence set forth in SEQ ID NO: 78, an FR3 sequence set forth in SEQ ID NO: 79, and an FR4 sequence set forth in SEQ ID NO: 73; or
(5) an FR1 sequence set forth in SEQ ID NO: 70, an FR2 sequence set forth in SEQ ID NO: 71, an FR3 sequence set forth in SEQ ID NO: 81, and an FR4 sequence set forth in SEQ ID NO: 73.

11. The bispecific antibody according to claim 7 or 8, wherein the second antigen-binding moiety is selected from:
(a) a sequence that has the amino acid sequence set forth in SEQ ID NO: 82 or 83 or 84 or 85 or 86 or 87 or 88 or 89 or 90 or 91; or
(b) an amino acid sequence that has 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more sequence identity compared to (a).

12. A bispecific antibody, comprising a first antigen-binding moiety and a second antigen-binding moiety, wherein:
the first antigen-binding moiety is capable of binding to GARP, TGF-β, and/or GAPR-TGF-β complex, and the second antigen-binding moiety is capable of binding to PD-L1;
the second antigen-binding moiety comprises a CDR1, a CDR2, and a CDR3, wherein:
(a) the CDR1 comprises a sequence selected from the group consisting of SEQ ID NOs: 60, 63, and 66, or sequences that have one or two amino acid substitutions, deletions, or additions compared to SEQ ID NOs: 60, 63, and 66;
(b) the CDR2 comprises a sequence selected from the group consisting of SEQ ID NOs: 61, 64, and 67, or sequences that have one or two amino acid substitutions, deletions, or additions compared to SEQ ID NOs: 61, 64, and 67;
(c) the CDR3 comprises a sequence selected from the group consisting of SEQ ID NOs: 62, 65, 68, and 69, or sequences that have one, two, or three amino acid substitutions, deletions, or additions compared to SEQ ID NOs: 62, 65, 68, and 69;
the CDR1, CDR2, and CDR3 sequences are obtained based on the IMGT definition scheme.

13. The bispecific antibody according to claim 12, wherein the second antigen-binding moiety comprises:
(1) a CDR1 sequence set forth in SEQ ID NO: 60, a CDR2 sequence set forth in SEQ ID NO: 61, and a CDR3 sequence set forth in SEQ ID NO: 62; or
(2) a CDR1 sequence set forth in SEQ ID NO: 63, a CDR2 sequence set forth in SEQ ID NO: 64, and a CDR3 sequence set forth in SEQ ID NO: 65; or
(3) a CDR1 sequence set forth in SEQ ID NO: 66, a CDR2 sequence set forth in SEQ ID NO: 67, and a CDR3 sequence set forth in SEQ ID NO: 68; or
(4) a CDR1 sequence set forth in SEQ ID NO: 60, a CDR2 sequence set forth in SEQ ID NO: 61, and a CDR3 sequence set forth in SEQ ID NO: 69.

14. The bispecific antibody according to claim 12 or 13, wherein the second antigen-binding moiety further comprises framework regions, including an FR1, an FR2, an FR3, and an FR4; the FR1, FR2, FR3, and FR4 comprise the following amino acid sequences:
(1) the FR1 comprises a sequence selected from the sequence set forth in SEQ ID NO: 70 or 74 or 77 or 80, or a sequence that has one, two, or three conservative amino acid substitutions compared to the sequence set forth in SEQ ID NO: 70 or 74 or 77 or 80;
(2) the FR2 comprises a sequence selected from the sequence set forth in SEQ ID NO: 71 or 75 or 78, or a sequence that has one, two, or three conservative amino acid substitutions compared to the sequence set forth in SEQ ID NO: 71 or 75 or 78;
(3) the FR3 comprises a sequence selected from the sequence set forth in SEQ ID NO: 72 or 76 or 79 or 81, or a sequence that has one, two, three, or four conservative amino acid substitutions compared to the sequence set forth in SEQ ID NO: 72 or 76 or 79 or 81;
(4) the FR4 is selected from the sequence set forth in SEQ ID NO: 73, or a sequence that has one or two conservative amino acid substitutions compared to the sequence set forth in SEQ ID NO: 73.

15. The bispecific antibody according to claim 12 or 13, wherein the second antigen moiety further comprises framework regions (FRs), and the framework regions (FRs) include:
(1) an FR1, which is the amino acid sequence set forth in SEQ ID NO: 70; an FR2, which is the amino acid sequence set forth in SEQ ID NO: 71; an FR3, which is the amino acid sequence set forth in SEQ ID NO: 72; and an FR4, which is the amino acid sequence set forth in SEQ ID NO: 73; or
(2) an FR1, which is the amino acid sequence set forth in SEQ ID NO: 74; an FR2, which is the amino acid sequence set forth in SEQ ID NO: 75; an FR3, which is the amino acid sequence set forth in SEQ ID NO: 76; and an FR4, which is the amino acid sequence set forth in SEQ ID NO: 73; or
(3) an FR1, which is the amino acid sequence set forth in SEQ ID NO: 77; an FR2, which is the amino acid sequence set forth in SEQ ID NO: 78; an FR3, which is the amino acid sequence set forth in SEQ ID NO: 79; and an FR4, which is the amino acid sequence set forth in SEQ ID NO: 73; or
(4) an FR1, which is the amino acid sequence set forth in SEQ ID NO: 80; an FR2, which is the amino acid sequence set forth in SEQ ID NO: 78; an FR3, which is the amino acid sequence set forth in SEQ ID NO: 79; and an FR4, which is the amino acid sequence set forth in SEQ ID NO: 73; or
(5) an FR1, which is the amino acid sequence set forth in SEQ ID NO: 70; an FR2, which is the amino acid sequence set forth in SEQ ID NO: 71; an FR3, which is the amino acid sequence set forth in SEQ ID NO: 81; and an FR4, which is the amino acid sequence set forth in SEQ ID NO: 73.

16. The bispecific antibody according to any of claims 12-15, wherein the second antigen-binding moiety is selected from:
(a) a sequence that has the amino acid sequence set forth in SEQ ID NO: 82 or 83 or 84 or 85 or 86 or 87 or 88 or 89 or 90 or 91; or
(b) an amino acid sequence that has 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more sequence identity compared to (a).

17. The bispecific antibody according to any of claims 12-16, wherein the first antigen-binding moiety is combined with a first heavy chain constant domain CH1, a light chain constant domain VL, and an Fc region to form an IgG-type structure;
the second antigen-binding moiety is linked to the IgG-type structure by a linker.

18. A bispecific antibody, comprising a first antigen-binding moiety and a second antigen-binding moiety, wherein the first antigen-binding moiety is capable of binding to TGF-β, GARP, or GARP-TGF-β complex, and the second antigen-binding moiety is capable of binding to PD-L1;
the first antigen-binding moiety comprises a heavy chain variable region and a light chain variable region; the heavy chain variable region comprises:
HCDR sequences set forth in SEQ ID NOs: 1, 2, and 3,
or HCDR sequences set forth in SEQ ID NOs: 4, 5, and 6,
or HCDR sequences set forth in SEQ ID NOs: 7, 8, and 9,
or HCDR sequences set forth in SEQ ID NOs: 10, 11, and 12,
or HCDR sequences set forth in SEQ ID NOs: 13, 14, and 15,
or HCDR sequences set forth in SEQ ID NOs: 16, 17, and 18;
the light chain variable region comprises:
LCDR sequences set forth in SEQ ID NOs: 19, 20, and 21,
or LCDR sequences set forth in SEQ ID NOs: 22, 23, and 24,
or LCDR sequences set forth in SEQ ID NOs: 25, 20, and 26,
or LCDR sequences set forth in SEQ ID NOs: 27, 28, and 29,
or LCDR sequences set forth in SEQ ID NOs: 30, 31, and 32,
or LCDR sequences set forth in SEQ ID NOs: 33, 34, and 35;
the second antigen-binding moiety comprises a CDR1, a CDR2, and a CDR3;
the CDR1 comprises the sequence set forth in SEQ ID NO: 60 or 63 or 66;
the CDR2 comprises the sequence set forth in SEQ ID NO: 61 or 64 or 67;
the CDR3 comprises the sequence set forth in SEQ ID NO: 62 or 65 or 68 or 69;
the HCDR sequences and the LCDR sequences, as well as the CDR1, CDR2, and CDR3 sequences, are obtained based on the IMGT definition scheme.

19. The bispecific antibody according to claim 18, wherein:
the first antigen-binding moiety has:
(1) HCDR sequences set forth in SEQ ID NOs: 1, 2, and 3 and LCDR sequences set forth in SEQ ID NOs: 19, 20, and 21; or
(2) HCDR sequences set forth in SEQ ID NOs: 4, 5, and 6 and LCDR sequences set forth in SEQ ID NOs: 22, 23, and 24; or
(3) HCDR sequences set forth in SEQ ID NOs: 7, 8, and 9 and LCDR sequences set forth in SEQ ID NOs: 25, 20, and 26; or
(4) HCDR sequences set forth in SEQ ID NOs: 10, 11, and 12 and LCDR sequences set forth in SEQ ID NOs: 27, 28, and 29; or
(5) HCDR sequences set forth in SEQ ID NOs: 13, 14, and 15 and LCDR sequences set forth in SEQ ID NOs: 30, 31, and 32; or
(6) HCDR sequences set forth in SEQ ID NOs: 16, 17, and 18 and LCDR sequences set forth in SEQ ID NOs: 33, 34, and 35;
the second antigen-binding moiety is selected from:
(1) a sequence that has: a CDR1, which is the amino acid sequence set forth in SEQ ID NO: 60; a CDR2, which is the amino acid sequence set forth in SEQ ID NO: 61; and a CDR3, which is the amino acid sequence set forth in SEQ ID NO: 62; or
(2) a sequence that has: a CDR1, which is the amino acid sequence set forth in SEQ ID NO: 63; a CDR2, which is the amino acid sequence set forth in SEQ ID NO: 64; and a CDR3, which is the amino acid sequence set forth in SEQ ID NO: 65; or
(3) a sequence that has: a CDR1, which is the amino acid sequence set forth in SEQ ID NO: 66; a CDR2, which is the amino acid sequence set forth in SEQ ID NO: 67; and a CDR3, which is the amino acid sequence set forth in SEQ ID NO: 68; or
(4) a sequence that has: a CDR1, which is the amino acid sequence set forth in SEQ ID NO: 60; a CDR2, which is the amino acid sequence set forth in SEQ ID NO: 61; and a CDR3, which is the amino acid sequence set forth in SEQ ID NO: 69;
the first antigen-binding moiety is combined with a first heavy chain constant domain CH1, a light chain constant domain VL, and an Fc region to form an IgG-type structure, and the second antigen-binding moiety is linked to the IgG-type structure by a linker.

20. The bispecific antibody according to claim 19, wherein the first antigen-binding moiety comprises:
(1) a heavy chain variable region set forth in SEQ ID NO: 36 or 37 or 38 or 39 or 40 or 41, and a light chain variable region set forth in SEQ ID NO: 48 or 49 or 50 or 51 or 52 or 53; or
(2) a heavy chain variable region set forth in SEQ ID NO: 42 or 43 or 44 or 45 or 46 or 47, and a light chain variable region set forth in SEQ ID NO: 54 or 55 or 56 or 57 or 58 or 59;
the second antigen-binding moiety comprises:
the amino acid sequence set forth in SEQ ID NO: 82 or 83 or 84 or 85 or 86 or 87 or 88 or 89 or 90 or 91.

21. A bispecific antibody, comprising a first antigen-binding moiety and a second antigen-binding moiety, wherein:
the first antigen-binding moiety comprises:
the HCDR1, HCDR2, and HCDR3 sequences of a heavy chain variable region set forth in SEQ ID NO: 36 or 37 or 38 or 39 or 40 or 41 or 42 or 43 or 44 or 45 or 46 or 47 and the LCDR1, LCDR2, and LCDR3 sequences of a light chain variable region set forth in SEQ ID NO: 48 or 49 or 50 or 51 or 52 or 53 or 54 or 55 or 56 or 57 or 58 or 59;
the first antigen-binding moiety is capable of binding to TGF-β, GARP, or GARP-TGF-β complex, and the second antigen-binding moiety is capable of binding to PD-L1.

22. The bispecific antibody according to claim 21, wherein the second antigen-binding moiety comprises:
the CDR1, CDR2, and CDR3 sequences of the amino acid set forth in SEQ ID NO: 82 or 83 or 84 or 85 or 86 or 87 or 88 or 89 or 90 or 91.

23. A bispecific antibody, comprising:
a first antigen-binding moiety that specifically binds to TGF-β, GARP, and/or GARP-TGF-β complex and a second antigen-binding moiety that specifically binds to PD-L1, wherein:
the bispecific antibody further comprises a first heavy chain constant domain CH1 and a light chain constant domain CL; the first antigen-binding moiety is covalently linked to the first heavy chain constant domain CH1 and the light chain constant domain CL;
the bispecific antibody further comprises an Fc region; the Fc region is linked to the first heavy chain constant domain CH1 by a hinge region, and the second antigen-binding moiety is linked to the Fc region and/or the light chain constant domain CL by a linker.

24. The bispecific antibody according to claim 23, being selected from at least one of the following:
(i) a bispecific antibody in which the second antigen-binding moiety is linked to the carboxy-terminus (C-terminus) of the Fc region by a linker;
(ii) a bispecific antibody in which the second antigen-binding moiety is linked to the carboxy-terminus (C-terminus) of the light chain constant domain by a linker; and
(iii) a bispecific antibody in which at least a portion of the second antigen-binding moiety is linked to the carboxy-terminus (C-terminus) of the Fc region by a first linker and at least a portion is linked to the carboxy-terminus (C-terminus) of the light chain constant domain by a second linker.

25. The bispecific antibody according to claim 23, having at least one of the following properties:
(a) specifically binding to PD-L1;
(b) specifically binding to glycoprotein A repetitions predominant (GARP);
(c) specifically binding to GARP-TGF-β complex;
(d) specifically binding to TGF-β;
(e) having inhibitory activity against the immunosuppressive function of regulatory T cells; and
(f) having anti-tumor activity.

26. A monoclonal antibody or an antigen-binding fragment, comprising a heavy chain variable region and a light chain variable region, wherein the heavy chain variable region and the light chain variable region comprise:
HCDR sequences set forth in SEQ ID NOs: 1, 2, and 3 or sequences that have one, two, or three amino acid substitutions, deletions, or additions compared to the HCDR sequences set forth in SEQ ID NOs: 1, 2, and 3, and LCDR sequences set forth in SEQ ID NOs: 19, 20, and 21 or sequences that have one, two, or three amino acid substitutions, deletions, or additions compared to the LCDR sequences set forth in SEQ ID NOs: 19, 20, and 21;
or HCDR sequences set forth in SEQ ID NOs: 4, 5, and 6 or sequences that have one, two, or three amino acid substitutions, deletions, or additions compared to the HCDR sequences set forth in SEQ ID NOs: 4, 5, and 6, and LCDR sequences set forth in SEQ ID NOs: 22, 23, and 24 or sequences that have one, two, or three amino acid substitutions, deletions, or additions compared to the LCDR sequences set forth in SEQ ID NOs: 22, 23, and 24;
or HCDR sequences set forth in SEQ ID NOs: 7, 8, and 9 or sequences that have one, two, or three amino acid substitutions, deletions, or additions compared to the HCDR sequences set forth in SEQ ID NOs: 7, 8, and 9, and LCDR sequences set forth in SEQ ID NOs: 25, 20, and 26 sequences that have one, two, or three amino acid substitutions, deletions, or additions;
or HCDR sequences set forth in SEQ ID NOs: 10, 11, and 12 or sequences that have one, two, or three amino acid substitutions, deletions, or additions compared to the HCDR sequences set forth in SEQ ID NOs: 10, 11, and 12, and LCDR sequences set forth in SEQ ID NOs: 27, 28, and 29 or sequences that have one, two, or three amino acid substitutions, deletions, or additions compared to the LCDR sequences set forth in SEQ ID NOs: 27, 28, and 29;
or HCDR sequences set forth in SEQ ID NOs: 13, 14, and 15 or sequences that have one, two, or three amino acid substitutions, deletions, or additions compared to the HCDR sequences set forth in SEQ ID NOs: 13, 14, and 15, and LCDR sequences set forth in SEQ ID NOs: 30, 31, and 32 or sequences that have one, two, or three amino acid substitutions, deletions, or additions compared to the LCDR sequences set forth in SEQ ID NOs: 30, 31, and 32;
or HCDR sequences set forth in SEQ ID NOs: 16, 17, and 18 or sequences that have one, two, or three amino acid substitutions, deletions, or additions compared to the HCDR sequences set forth in SEQ ID NOs: 16, 17, and 18, and LCDR sequences set forth in SEQ ID NOs: 33, 34, and 35 or sequences that have one, two, or three amino acid substitutions, deletions, or additions compared to the LCDR sequences set forth in SEQ ID NOs: 33, 34, and 35;
the HCDR sequences and the LCDR sequences are obtained based on the IMGT definition scheme.

27. A monoclonal antibody or an antigen-binding fragment, comprising a heavy chain variable region and a light chain variable region, wherein the heavy chain variable region and the light chain variable region have:
(1) HCDR sequences set forth in SEQ ID NOs: 1, 2, and 3 and LCDR sequences set forth in SEQ ID NOs: 19, 20, and 21; or
(2) HCDR sequences set forth in SEQ ID NOs: 4, 5, and 6 and LCDR sequences set forth in SEQ ID NOs: 22, 23, and 24; or
(3) HCDR sequences set forth in SEQ ID NOs: 7, 8, and 9 and LCDR sequences set forth in SEQ ID NOs: 25, 20, and 26; or
(4) HCDR sequences set forth in SEQ ID NOs: 10, 11, and 12 and LCDR sequences set forth in SEQ ID NOs: 27, 28, and 29; or
(5) HCDR sequences set forth in SEQ ID NOs: 13, 14, and 15 and LCDR sequences set forth in SEQ ID NOs: 30, 31, and 32; or
(6) HCDR sequences set forth in SEQ ID NOs: 16, 17, and 18 and LCDR sequences set forth in SEQ ID NOs: 33, 34, and 35.

28. A monoclonal antibody or an antigen-binding fragment, comprising a heavy chain variable region and a light chain variable region, wherein the heavy chain variable region comprises complementarity-determining regions HCDR1, HCDR2, and HCDR3, and the light chain variable region comprises complementarity-determining regions LCDR1, LCDR2, and LCDR3, wherein:
the HCDR1 comprises the sequence set forth in SEQ ID NO: 1 or 4 or 7 or 10 or 13 or 16;
the HCDR2 comprises the sequence set forth in SEQ ID NO: 2 or 5 or 8 or 11 or 14 or 17;
the HCDR3 comprises the sequence set forth in SEQ ID NO: 3 or 6 or 9 or 12 or 15 or 18;
the LCDR1 comprises the sequence set forth in SEQ ID NO: 19 or 22 or 25 or 27 or 30 or 33;
the LCDR2 comprises the sequence set forth in SEQ ID NO: 20 or 23 or 28 or 31 or 34;
the LCDR3 comprises the sequence set forth in SEQ ID NO: 21 or 24 or 26 or 29 or 32 or 35.

29. A monoclonal antibody or an antigen-binding fragment, comprising:
the HCDR1, HCDR2, and HCDR3 sequences of a heavy chain variable region set forth in SEQ ID NO: 36 or 37 or 38 or 39 or 40 or 41 or 42 or 43 or 44 or 45 or 46 or 47 and the LCDR1, LCDR2, and LCDR3 sequences of a light chain variable region set forth in SEQ ID NO: 48 or 49 or 50 or 51 or 52 or 53 or 54 or 55 or 56 or 57 or 58 or 59.

30. The monoclonal antibody or the antigen-binding fragment according to any of claims 26-29, comprising:
a heavy chain variable region that has at least 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence set forth in SEQ ID NO: 36 or 37 or 38 or 39 or 40 or 41 or 42 or 43 or 44 or 45 or 46 or 47, and
a light chain variable region that has at least 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence set forth in SEQ ID NO: 48 or 49 or 50 or 51 or 52 or 53 or 54 or 55 or 56 or 57 or 58 or 59.

31. The monoclonal antibody or the antigen-binding fragment according to any of claims 26-30, selected from:
a heavy chain variable region set forth in SEQ ID NO: 36 and a light chain variable region set forth in SEQ ID NO: 48; or
a heavy chain variable region set forth in SEQ ID NO: 37 and a light chain variable region set forth in SEQ ID NO: 49; or
a heavy chain variable region set forth in SEQ ID NO: 38 and a light chain variable region set forth in SEQ ID NO: 50; or
a heavy chain variable region set forth in SEQ ID NO: 39 and a light chain variable region set forth in SEQ ID NO: 51; or
a heavy chain variable region set forth in SEQ ID NO: 40 and a light chain variable region set forth in SEQ ID NO: 52; or
a heavy chain variable region set forth in SEQ ID NO: 41 and a light chain variable region set forth in SEQ ID NO: 53; or
a heavy chain variable region set forth in SEQ ID NO: 42 and a light chain variable region set forth in SEQ ID NO: 54; or
a heavy chain variable region set forth in SEQ ID NO: 43 and a light chain variable region set forth in SEQ ID NO: 55; or
a heavy chain variable region set forth in SEQ ID NO: 44 and a light chain variable region set forth in SEQ ID NO: 56; or
a heavy chain variable region set forth in SEQ ID NO: 45 and a light chain variable region set forth in SEQ ID NO: 57; or
a heavy chain variable region set forth in SEQ ID NO: 46 and a light chain variable region set forth in SEQ ID NO: 58; or
a heavy chain variable region set forth in SEQ ID NO: 47 and a light chain variable region set forth in SEQ ID NO: 59.

32. The monoclonal antibody or the antigen-binding fragment according to any of claims 26-31, further comprising an Fc region.

33. A polynucleotide, wherein the polynucleotide encodes the bispecific antibody according to any of claims 1-25, or the monoclonal antibody or the antigen-binding fragment according to any of claims 26-32.

34. A construct, comprising the polynucleotide according to claim 33.

35. A host cell, comprising the polynucleotide according to claim 33 or the construct according to claim 34.

36. A pharmaceutical composition, comprising:
the bispecific antibody according to any of claims 1-25, or the monoclonal antibody or the antigen-binding fragment according to any of claims 26-32; and
a pharmaceutically acceptable carrier.

37. An antibody conjugate, comprising:
the bispecific antibody according to any of claims 1-25, or the monoclonal antibody or the antigen-binding fragment according to any of claims 26-32; and
a functional small molecule linked to the bispecific antibody or the monoclonal antibody or the antigen-binding fragment.

38. A kit, wherein the kit comprises the bispecific antibody according to any of claims 1-25, or the monoclonal antibody or the antigen-binding fragment according to any of claims 26-32.

39. A method for producing the bispecific antibody according to any of claims 1-25, or the monoclonal antibody or the antigen-binding fragment according to any of claims 26-32, comprising:
culturing the host cell according to claim 35, and
collecting the bispecific antibody or the monoclonal antibody or the antigen-binding fragment from the culture.

40. A method for preventing and/or treating a disease, comprising:
administering to a subject in need thereof an effective amount of the bispecific antibody according to any of claims 1-25, or the monoclonal antibody or the antigen-binding fragment according to any of claims 26-32, or the pharmaceutical composition according to claim 36, or the antibody conjugate according to claim 37.

41. The method according to claim 40, wherein the disease is selected from cancer or an autoimmune disease.

42. Use of the bispecific antibody according to any of claims 1-25, or the monoclonal antibody or the antigen-binding fragment according to any of claims 26-32, in the preparation of a medicament or a kit or an antibody conjugate.
